(19) Europäisches Patentamt
European Patent Office
Office européen des brevets

(11) **EP 4 282 420 A1**

(12) **EUROPEAN PATENT APPLICATION**
published in accordance with Art. 153(4) EPC

(43) Date of publication:
**29.11.2023 Bulletin 2023/48**

(21) Application number: **22742650.9**

(22) Date of filing: **20.01.2022**

(51) International Patent Classification (IPC):
***A61K 31/7064*** *(2006.01)* ***A61P 31/12*** *(2006.01)*

(52) Cooperative Patent Classification (CPC):
**A61K 31/7064; A61P 31/12;** Y02A 50/30

(86) International application number:
**PCT/JP2022/001997**

(87) International publication number:
**WO 2022/158528 (28.07.2022 Gazette 2022/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.01.2021 JP 2021007531**

(71) Applicant: **National University Corporation Hokkaido University**
**Sapporo-shi, Hokkaido 060-0808 (JP)**

(72) Inventors:
- **MAENAKA Katsumi**
  **Sapporo-shi, Hokkaido 060-0808 (JP)**
- **MATSUDA Akira**
  **Sapporo-shi, Hokkaido 060-0808 (JP)**
- **SAWA Hirofumi**
  **Sapporo-shi, Hokkaido 060-0808 (JP)**
- **ORBA Yasuko**
  **Sapporo-shi, Hokkaido 060-0808 (JP)**
- **SASAKI Michihito**
  **Sapporo-shi, Hokkaido 060-0808 (JP)**
- **UEMURA Kentaro**
  **Sapporo-shi, Hokkaido 060-0808 (JP)**

(74) Representative: **Dehns**
**St. Bride's House**
**10 Salisbury Square**
**London EC4Y 8JD (GB)**

(54) **ANTI-VIRAL AGENT**

(57) [Problem] The present invention has as an object to provide an antiviral agent effective as a remedy for COVID-19 and other viral infections.

[Resolution Means] An antiviral agent, made of: the compound represented by general formula (1) or (2) below [$R^1$ being $-(CH_2)n_1-Z^1-R^{11}$ or $-CH-(-Z^1-R^{11})_2$ ($n_1$ being 0 or 1; $Z^1$ being a single bond, -O-, -NH-, -S-, -SO-, $-SO_2-$, -CO-, -CO-O-, or -CH=N-O-; $R^{11}$ being a hydrogen atom, an alkyl group having 1 to 6 carbons, $-NR^{12}R^{13}$, $-N_3$, $-NO_2$, -CN, $-CH_2-CO-O-R^{14}$, or a five- or six-membered heterocyclic group including a nitrogen atom; $R^{12}$ and $R^{13}$ each independently being a hydrogen atom or an alkyl group having 1 to 6 carbons; and $R^{14}$ being a hydrogen atom or an alkyl group having 1 to 6 carbons], a derivative thereof, a salt of these compounds, or a solvate thereof.

EP 4 282 420 A1

[Chem. 1]

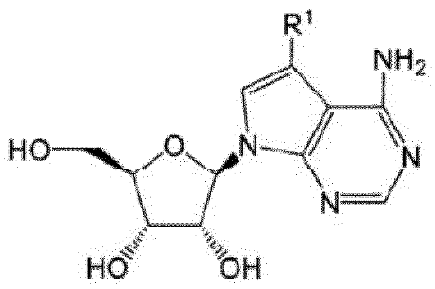

(1)

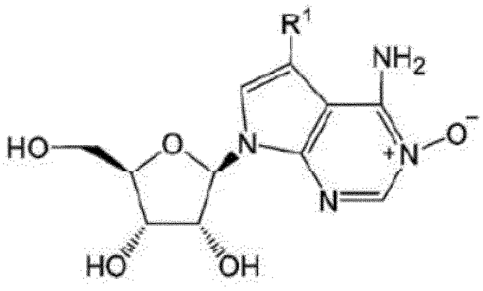

(2)

[FIG. 1]

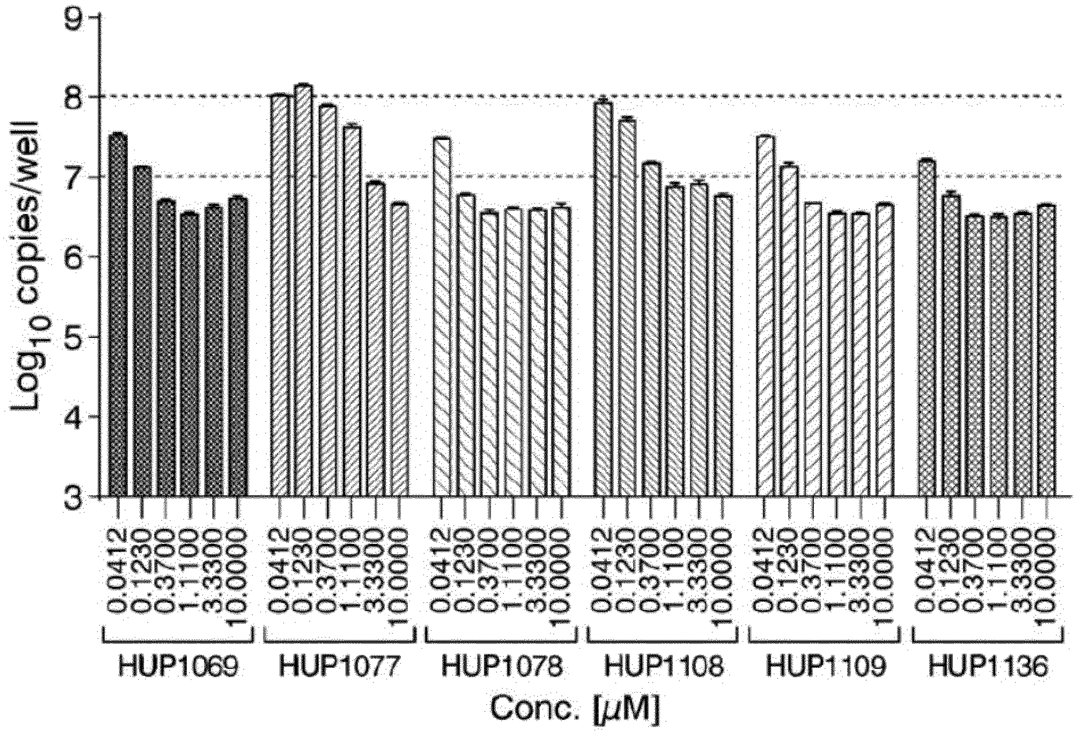

## Description

[Field of Art]

The present invention relates to an antiviral agent effective as a therapeutic agent for a virus such as a coronavirus or a flavivirus.

[Background Art]

Coronaviruses are enveloped viruses having positive-strand single-strand RNA as genes. There are coronaviruses infectious to humans that cause acute respiratory syndromes in addition to viruses that cause the common cold. For example, there is SARS-CoV, the cause of severe acute respiratory syndrome (SARS) that became widespread in 2003; MERS-CoV, the cause of Middle East respiratory syndrome (MERS) that became widespread in 2013; and SARS-CoV-2, the cause of the novel coronavirus disease COVID-19 that became widespread starting in 2019. For COVID-19 in particular, remedies are still few, and the development of more effective remedies is being sought.
Remdesivir exists as a remedy for COVID-19. Remdesivir (GS-5734) is a monophosphoramidate prodrug of GS-441524 and is an antiviral drug made of a nucleic-acid compound developed as a remedy for Ebola hemorrhagic fever and Marburg virus disease. It has antiviral activity against single-strand RNA viruses. GS-441524 is also anticipated to have a therapeutic effect against COVID-19 (non-patent literature 1). GS-441524 is an adenosine analog and is also the parent nucleoside of remdesivir.
Non-patent literature 5 reports that GS-441524 has antiviral activity against flaviviruses and coronaviruses.
Non-patent literature 6 reports that GS-621763 and MPV (molnupiravir) have a therapeutic effect on SARS-CoV-2 infections. GS-621763 is a prodrug of GS-441524, and MPV is a prodrug of NHC (N-hydroxycytidine).

[Prior-Art Literature]

[Non-Patent Literature]

[Non-Patent Literature 1] Yan and Muller, ACS Medicinal Chemistry Letters, 2020, vol. 11, p. 1361-1366.
[Non-Patent Literature 2] Watanabe and Ueda, Nucleosides & Nucleotides, 1983, vol. 2(2), p. 113-125.
[Non-Patent Literature 3] Bergstrom et al., Journal of Organic Chemistry, 1981, vol. 46(7), p. 1423-1431.
[Non-Patent Literature 4] Uematsu and Suhadolnik, Journal of Medicinal Chemistry, 1973, vol. 16(12), p. 1405-1407.
[Non-Patent Literature 5] Cho et al., Bioorganic & Medicinal Chemistry Letters, 22(2012), 2705-2707.
[Non-Patent Literature 6] Schafer et al., bioRxiv preprint doi: https://doi.ora/10.1101/2021.09.13.460111, Version 9.4, 2021.9.13.
[Non-Patent Literature 7] Rasmussen et al., Viruses 2021, 13(7), 1369.


[Summary of Invention]

[Problem to Be Solved by Invention]

The present invention has as an object to provide an antiviral agent effective as a remedy for COVID-19 and other viral infections.

[Means for Solving Problem]

The present inventors used a library, possessed by the Center for Research and Education on Drug Discovery in the Faculty of Pharmaceutical Sciences at Hokkaido University, of compounds having a nucleic-acid compound as a backbone to screen for compounds having antiviral activity against the novel coronavirus SARS-CoV-2 and two types of human coronaviruses (strains 229E and OC43) and selected compounds having high antiviral activity, thereby completing the present invention.
That is, the present invention provides the following antiviral agents and the like.

[1] An antiviral agent, made of: the compound represented by general formula (1) or (2) below-

[Chem. 1]

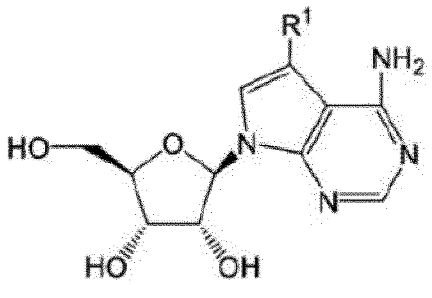

(1)

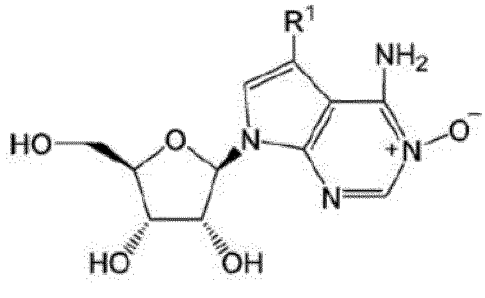

(2)

[in the formulas, $R^1$ being $-(CH_2)n_1-Z^1-R^{11}$ or $-CH-(-Z^1-R^{11})_2$ ($n_1$ being 0 or 1; $Z^1$ being a single bond, an oxygen atom, -NH-, a sulfur atom, -SO-, $-SO_2-$, -CO-, -CO-O-, or -CH=NO-; $R^{11}$ being a hydrogen atom, an alkyl group having 1 to 6 carbons, $-NR^{12}R^{13}$, $-N_3$, $-NO_2$, -CN, $-CH_2-CO-O-R^{14}$, or a five- or six-membered heterocyclic group including a nitrogen atom; $R^{12}$ and $R^{13}$ each independently being a hydrogen atom or an alkyl group having 1 to 6 carbons; and $R^{14}$ being a hydrogen atom or an alkyl group having 1 to 6 carbons-however, the nitrogen atom in the heterocyclic group and $Z^1$ being joined)]-a derivative thereof, a salt of these compounds, or a solvate thereof.

[2] The antiviral agent of [1], wherein general formula (1) is any among general formulas (1-1) to (1-15) below

[in the formulas, $R^{21}$ being a hydrogen atom, an alkyl group having 1 to 6 carbons, $-CH_2-COOH$, or an amino group; $R^{22}$ being a hydrogen atom, an alkyl group having 1 to 6 carbons, or an amino group; $R^{23}$, $R^{26}$, $R^{27}$, $R^{31}$, $R^{32}$, and $R^{33}$ being a hydrogen atom or an alkyl group having 1 to 6 carbons; $R^{24}$, $R^{25}$, $R^{29}$, $R^{30}$, and $R^{35}$ being an alkyl group having 1 to 6 carbons; $R^{28}$ being a hydrogen atom, an alkyl group having 1 to 6 carbons, or a cyano group; $R^{34}$ being a hydrogen atom, an alkyl group having 1 to 6 carbons, $-CH_2-N_3$, a cyano group, an amino group, a nitro group, or an azide group; and A being a five- or six-membered heterocyclic group including a nitrogen atom]

and general formula (2) is any among general formulas (2-1) to (2-15) below [in the formulas, $R^{21}$ to $R^{35}$ and A being as above].

[Chem. 2]

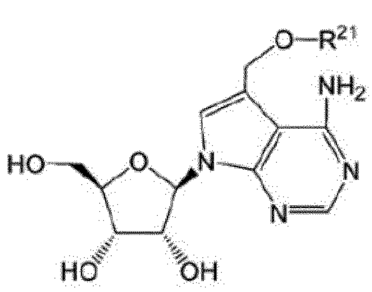

(1-1)

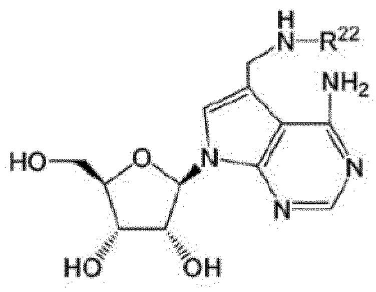

(1-2)

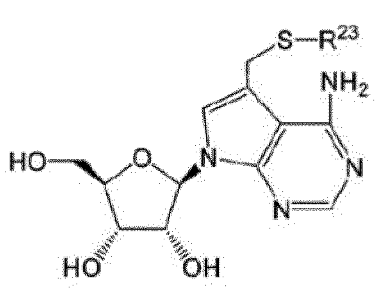

(1-3)

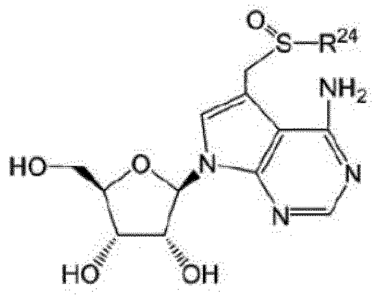

(1-4)

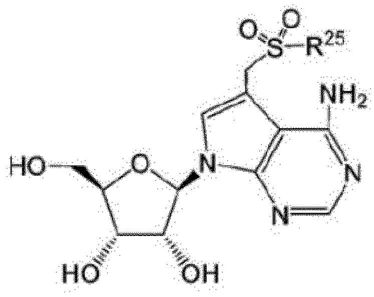

(1-5)

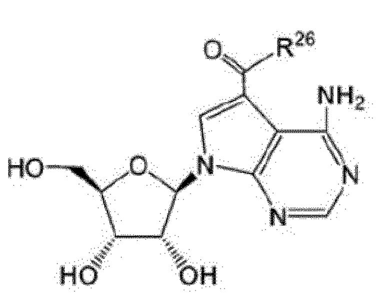

(1-6)

[Chem. 3]

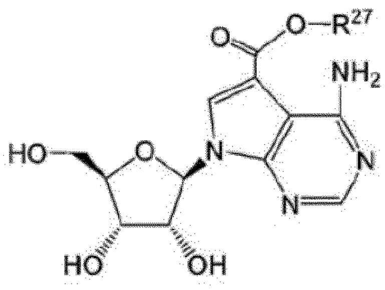

(1-7)

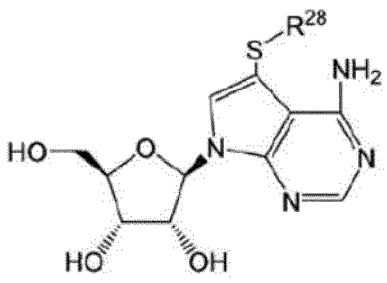

(1-8)

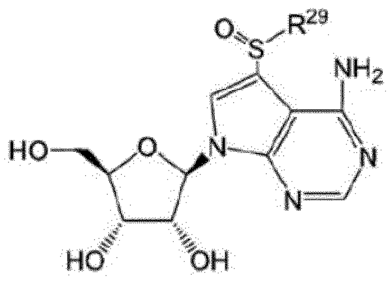

(1-9)

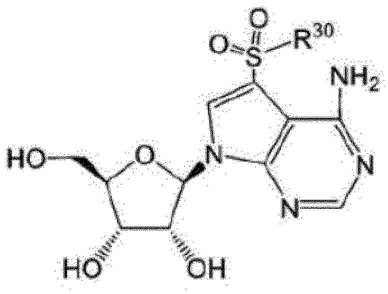

(1-10)

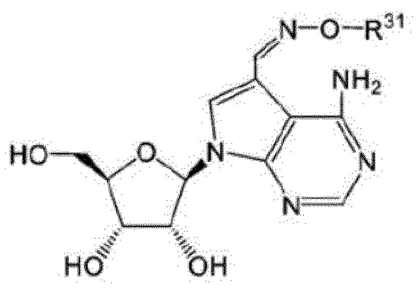

(1-11)

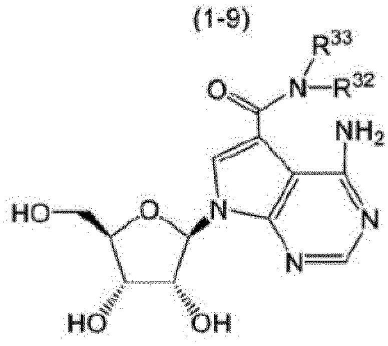

(1-12)

[Chem. 4]

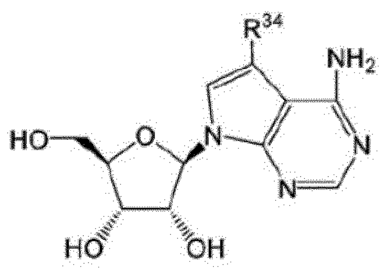

(1-13)

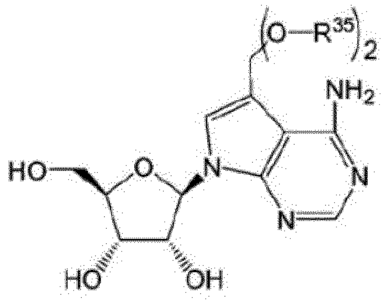

(1-14)

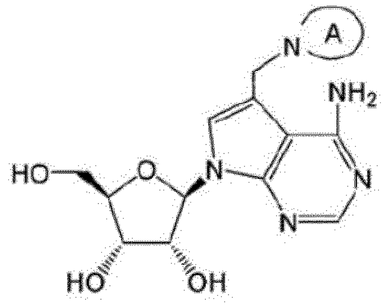

(1-15)

[Chem. 5]

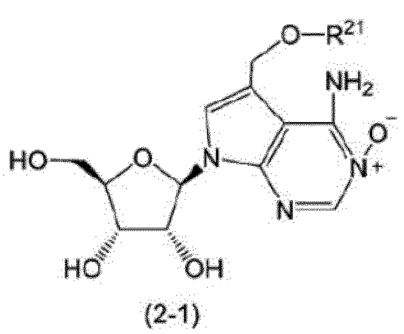
(2-1)

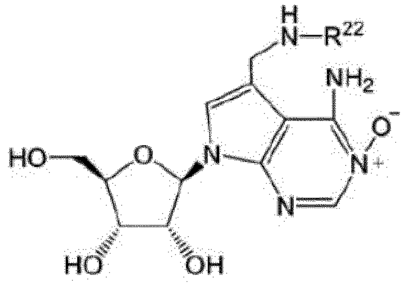
(2-2)

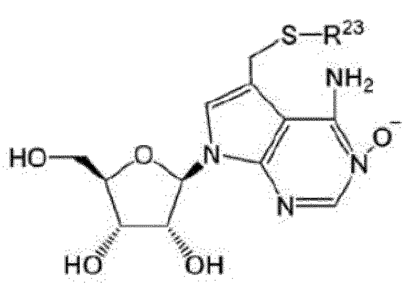
(2-3)

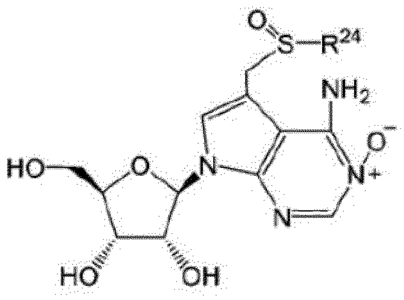
(2-4)

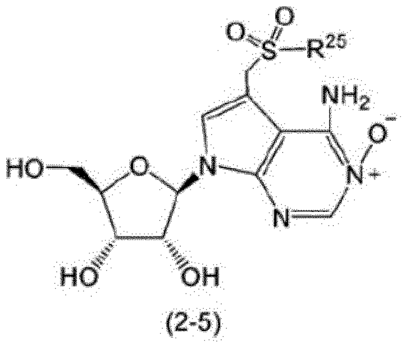
(2-5)

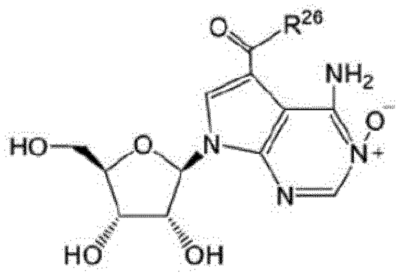
(2-6)

[Chem. 6]

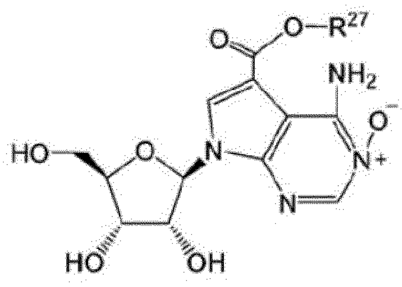
(2-7)

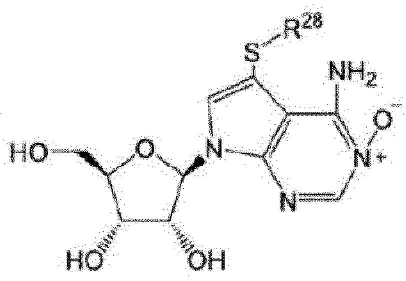
(2-8)

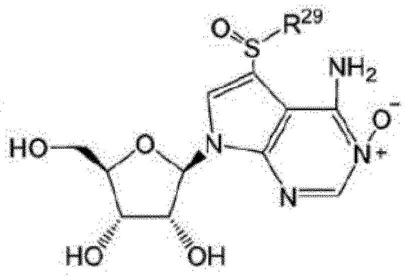
(2-9)

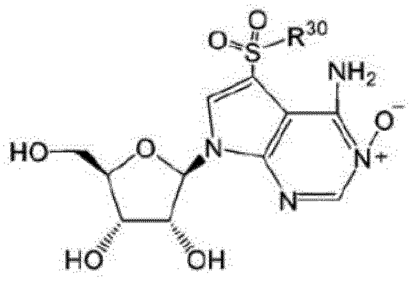
(2-10)

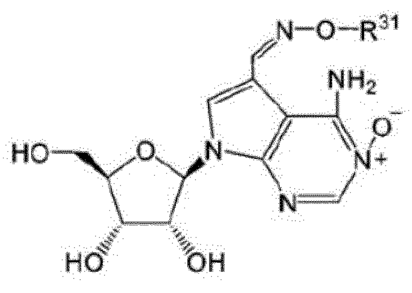
(2-11)

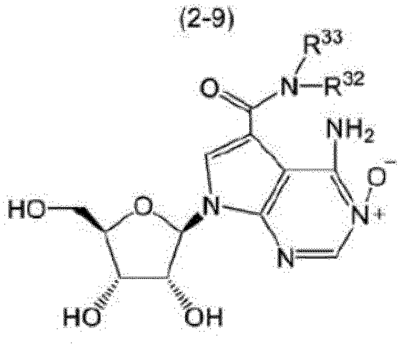
(2-12)

[Chem. 7]

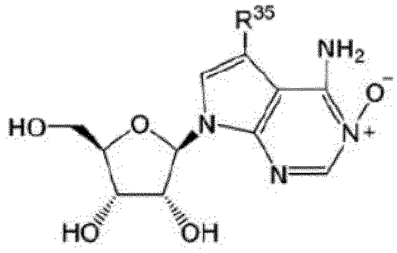

(2-13)

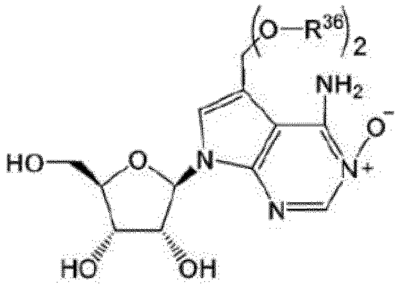

(2-14)

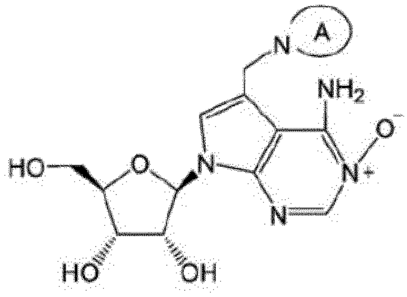

(2-15)

[3] The antiviral agent of [1] or [2], wherein the antiviral agent is made of one or more compounds selected from a group consisting of HUP1136, HUP1108, HUP1069, HUP1077, HUP1078, and HUP1109.

[Chem. 8]

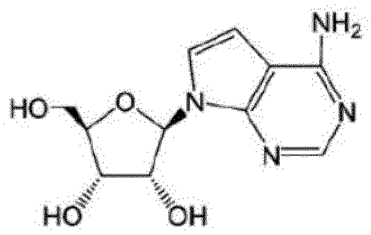

HUP1136

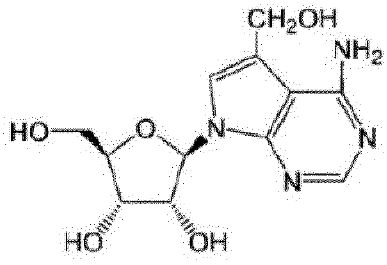

HUP1108

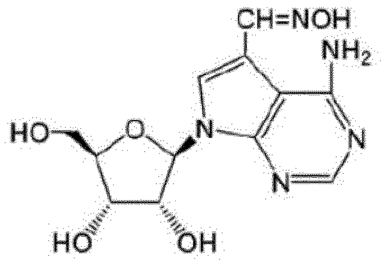

HUP1069

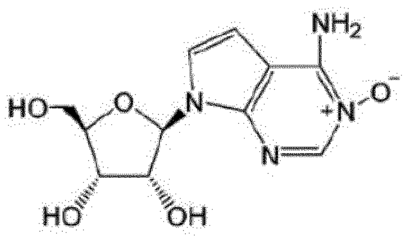

HUP1077

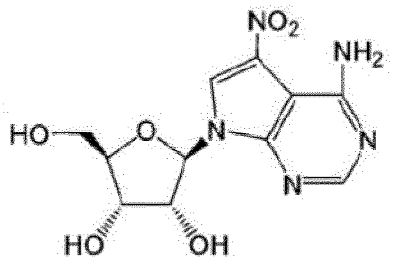

HUP1078

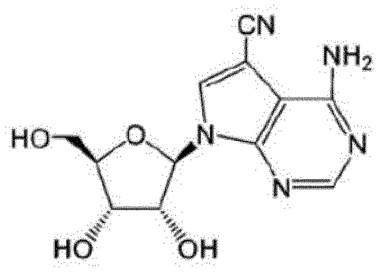

HUP1109

[4] A pharmaceutical composition whose active ingredient is the antiviral agent of any among [1] to [3].

[5] The pharmaceutical composition of [4], wherein the pharmaceutical composition is used to treat an infection due to a coronavirus or a flavivirus.

[6] The pharmaceutical composition of [5], wherein the pharmaceutical composition is used to treat COVID-19.

[7] The pharmaceutical composition of any one of [4] to [6], wherein the pharmaceutical composition is used for combination therapy with an inhibitor of 3CL protease.

[8] The pharmaceutical composition of [7], wherein the inhibitor of 3CL protease is at least one type selected from the group consisting of PF-07304814 and S-217622.

[9] A treatment method of treating a coronavirus infection or a flavivirus infection by administering an effective amount of the antiviral agent of any one among [1] to [3].

[10] The treatment method of [9], wherein an effective amount of an antiviral agent composed of HUP1108 is

administered

[11] The treatment method of [9] or [10], wherein the effective amount is less than 60 mg/kg.

[12] The treatment method of [11], wherein the effective amount is 20 mg/kg.

[13] The treatment method of any one among [9] to [12], wherein the coronavirus is a SARS-CoV-2.

[14] The treatment method of [13], wherein the SARS-CoV-2 is at least one selected from the group consisting of WK-521 strain, α strain, β strain, γ strain, δ strain, and o strain.

[15] The treatment method of any one of 9 to 12, wherein the flavivirus is at least one type selected from a group consisting of the dengue virus (DENV), the Zika virus (ZIKV), the yellow fever virus (YFV), the West Nile virus (WNV), and the Japanese encephalitis virus (JEV).

[16] The treatment method of any one among [9] to [15], wherein the inhibitor of 3CL protease is administered in combination.

[17] The treatment method of [16], wherein the inhibitor of 3CL protease is at least one selected from the group consisting of PF-07304814 and S-217622.

[18] A therapeutic combination of the antiviral agent of any one of [1] to [3] and an inhibitor of 3CL protease.

[19] The combination of [18], wherein the inhibitor of the 3CL protease is at least one selected from the group consisting of PF-07304814 and S-217622.

[20] The antiviral agent of any one among [1] to [3], wherein the antiviral agent is used to treat a coronavirus infection or a flavivirus infection.

[21] The antiviral agent of [20], wherein the coronavirus is at least one type selected from a group consisting of SARS-CoV-2 WK-521 strain, α strain, β strain, γ strain, δ strain, and o strain, and
the flavivirus is at least one type selected from a group consisting of dengue virus (DENV), the Zika virus (ZIKV), the yellow fever virus (YFV), the West Nile virus (WNV), and the Japanese encephalitis virus (JEV).

[Effects of Invention]

The antiviral agent of the present invention has as an active ingredient a nucleic-acid compound having high antiviral activity against an enveloped virus having positive-strand single-strand RNA as genes, such as a coronavirus or a flavivirus. As such, a pharmaceutical composition whose active ingredient is this antiviral agent is suitable as an *in vivo* or ex *vivo* antiviral agent and is very useful as an active ingredient of a pharmaceutical composition used to treat or prevent an infection due to a coronavirus or a flavivirus.

[Brief Description of Drawings]

[FIG. 1] A diagram illustrating measurement results of a viral-RNA amount ($\log_{10}$ [number of viral-RNA copies]) in a situation wherein in example 2, Caco-2 cells infected using SARS-CoV-2 are treated by any among compounds HUP1136, HUP1108, HUP1069, HUP1077, HUP1078, and HUP1109.
[FIG. 2] A diagram illustrating measurement results of a viral-RNA replication inhibition rate ([number of viral-RNA copies from cells treated by test samples]/[number of viral-RNA copies from cells of group having no test samples added thereto]×100%) in a situation wherein in example 2, Caco-2 cells infected using SARS-CoV-2 are treated by any among compounds HUP1136, HUP1108, HUP1069, HUP1077, HUP1078, and HUP1109.
[FIG. 3] A diagram illustrating measurement results of a relative viral-RNA amount in a situation wherein in example 3, Caco-2 cells infected using SARS-CoV-2 are treated by HUP1108 at three timings (Whole, Entry, and Post-Entry).
[FIG. 4] A diagram illustrating measurement results of a viral titer (logarithm values of $TCID_{50}$) in a situation wherein in example 3, Caco-2 cells infected using SARS-CoV-2 are treated by HUP1108 at three timings (Whole, Entry, and Post-Entry).

[FIG. 5] A diagram illustrating measurement results of a viral-RNA amount ($log_{10}$ [number of viral-RNA copies]) in a situation wherein in example 5, Caco-2 cells infected using SARS-CoV-2 (strain WK-521) and various variants thereof are treated by the compound HUP1108.

[FIG. 6] A diagram illustrating results of measuring an intrapulmonary viral titer of mice infected by SARS-CoV-2 and administered with HUP1108 and of mice infected by SARS-CoV-2 and not administered with HUP1108, in Embodiment 6.

[FIG. 7] A diagram illustrating results of measuring a survival rate of mice infected by SARS-CoV-2 and administered with HUP1108 and of mice infected by SARS-CoV-2 and not administered with HUP1108, in Embodiment 7.

[Embodiments of Invention]

In the present invention and the specification of the present application, "$C_{y-z}$ (y and z being positive integers that satisfy y < z)" signifies that a number of carbons is no less than y and no greater than z.

An antiviral agent of the present invention is made of the compound represented by general formula (1) below (also "compound (1)" hereinbelow) or the compound represented by general formula (2) below (also "compound (2)" hereinbelow).

[Chem. 9]

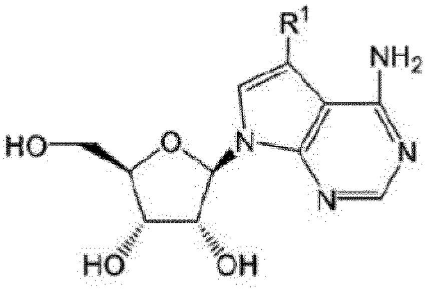

(1)

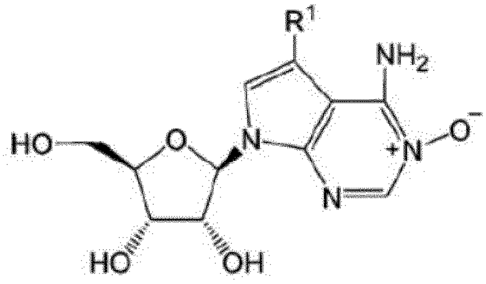

(2)

In general formula (1) and general formula (2), $R^1$ is a group represented by - $(CH_2)n1-Z^1-R^{11}$ or $-CH-(-Z^1-R^{11})_2$. Here, $n_1$ is 0 or 1, and $Z^1$ is a single bond, an oxygen atom, -NH-, a sulfur atom, -SO-, $-SO_2-$, -CO-, -CO-O-, or -CH=N-O-. When $n_1$ is 0, - $(CH_2)_0$- is a single bond and signifies that $Z^1$ is directly bonded to a carbon atom of a purine ring.

$R^{11}$ is a hydrogen atom, an alkyl group having 1 to 6 carbons, $-NR^{12}R^{13}$, $-N_3$, $-NO_2$, -CN, $-CH_2-CO-O-R^{14}$, or a five- or six-membered heterocyclic group including a nitrogen atom. $R^{12}$ and $R^{13}$ are each independently a hydrogen atom or an alkyl group having 1 to 6 carbons, and $R^{14}$ is a hydrogen atom or an alkyl group having 1 to 6 carbons. However, the nitrogen atom in the heterocyclic group is joined to $Z^1$.

When $R^{11}$, $R^{12}$, $R^{13}$, and $R^{14}$ are an alkyl group having 1 to 6 carbons, this alkyl group may be linear or branched. More specifically, an alkyl group having 1 to 6 carbons such as a methyl group, an ethyl group, an n-propyl group, an isopropyl group, an n-butyl group, a sec-butyl group, an isobutyl group, a tert-butyl group, an n-pentyl group, a sec-pentyl group, an isopentyl group, an n -hexyl group, a sec-hexyl group, or an isohexyl group is preferable; an alkyl group having 1 to 4 carbons is more preferable; an alkyl group having 1 to 3 carbons is further preferable; and a methyl group or an ethyl group is even further preferable.

More specifically, as compound (1), the compounds represented by general formulas (1-1) to (1-15) can be mentioned, and as compound (2), the compounds represented by general formulas (2-1) to (2-15) can be mentioned.

[Chem. 10]

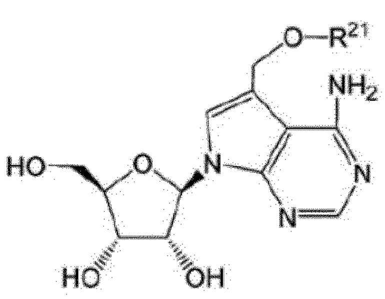
(1-1)

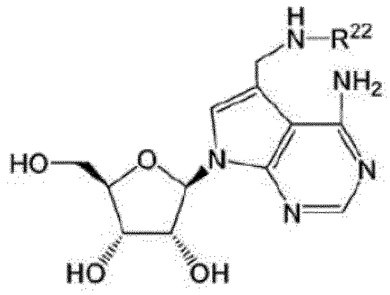
(1-2)

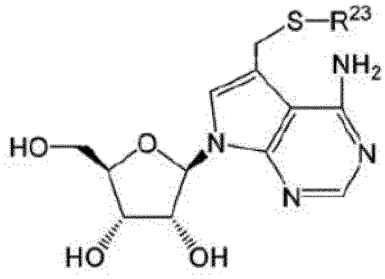
(1-3)

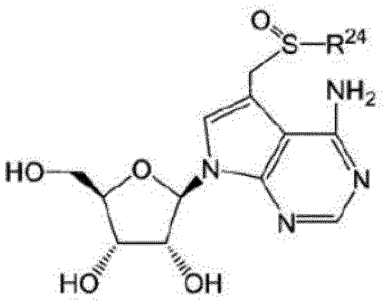
(1-4)

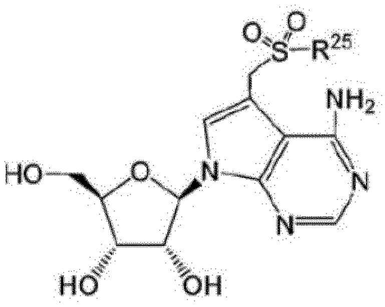
(1-5)

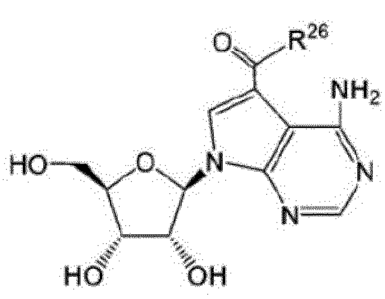
(1-6)

[Chem. 11]

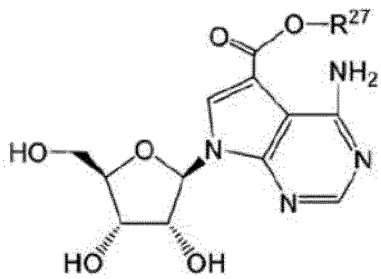
(1-7)

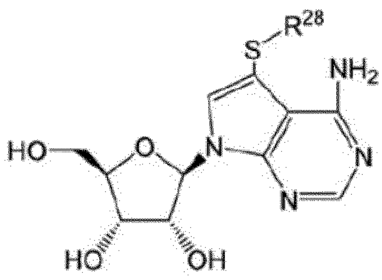
(1-8)

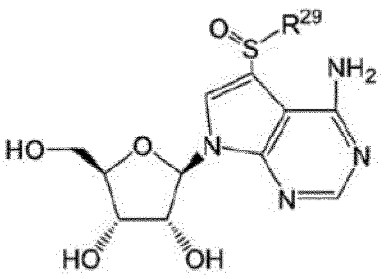
(1-9)

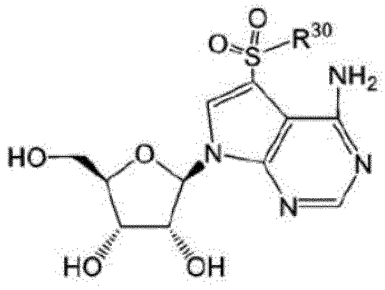
(1-10)

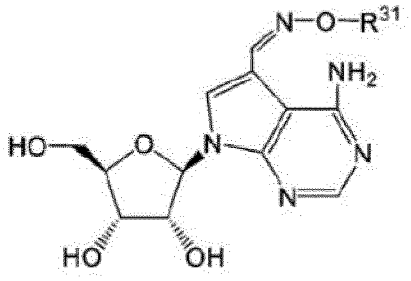
(1-11)

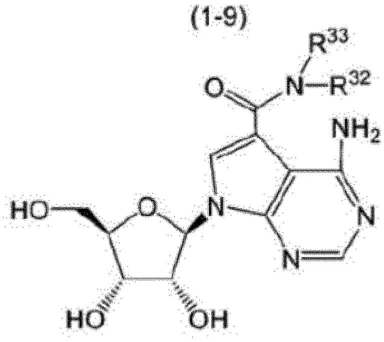
(1-12)

[Chem. 12]

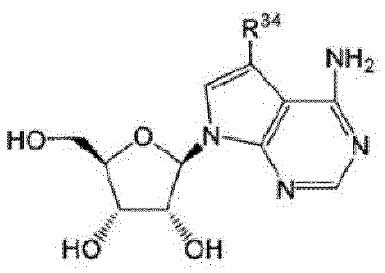

(1-13)

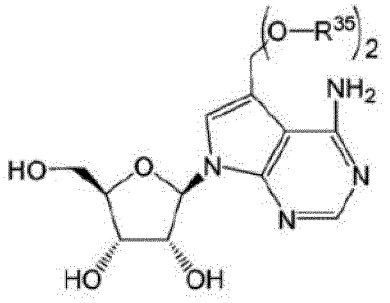

(1-14)

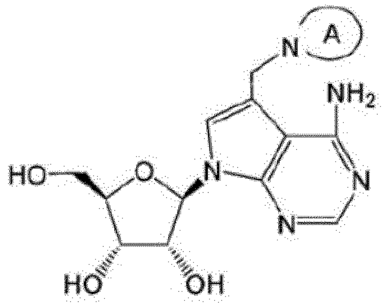

(1-15)

[Chem. 13]

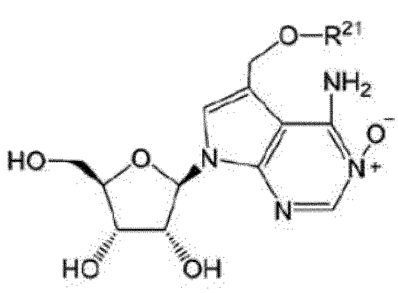

(2-1)

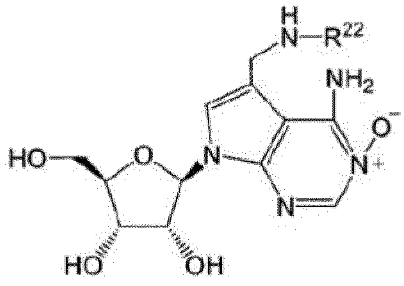

(2-2)

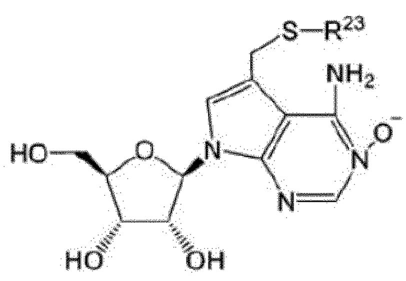

(2-3)

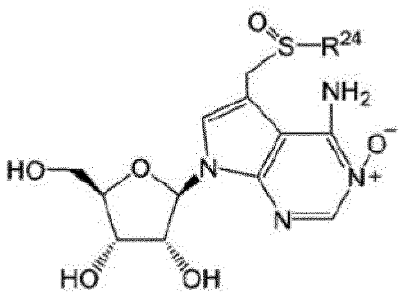

(2-4)

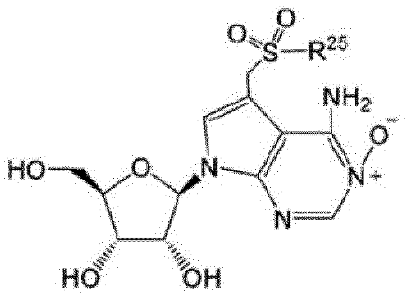

(2-5)

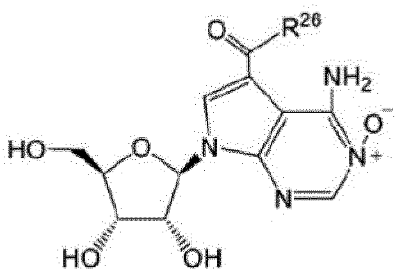

(2-6)

[Chem. 14]

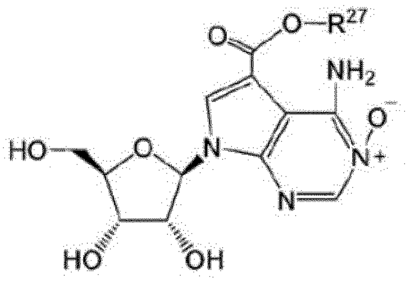

(2-7)

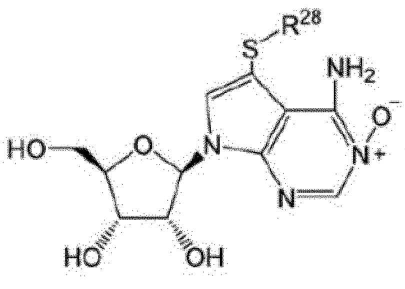

(2-8)

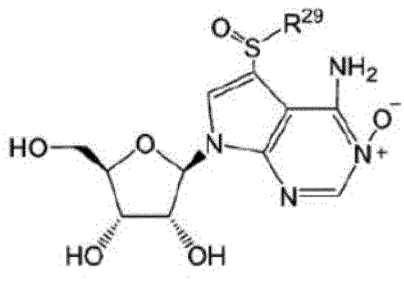

(2-9)

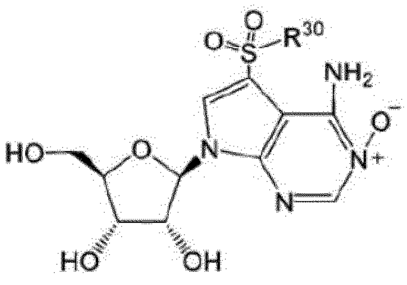

(2-10)

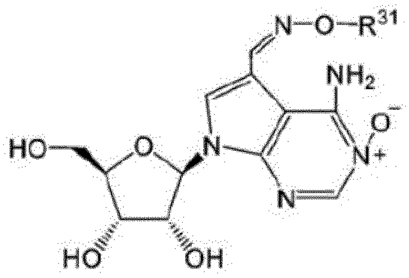

(2-11)

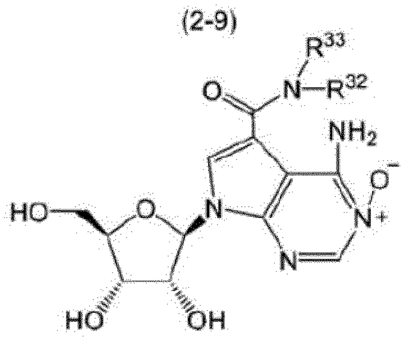

(2-12)

[Chem. 15]

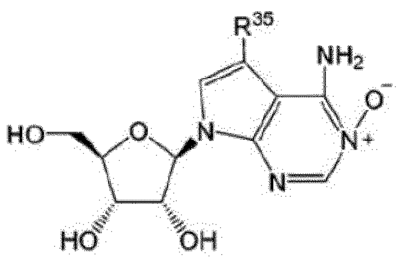

(2-13)

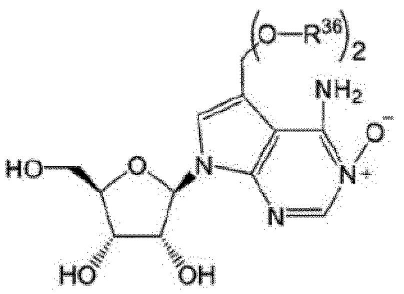

(2-14)

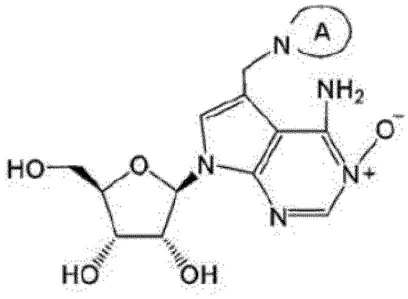

(2-15)

In general formula (1-1) and general formula (2-1), $R^{21}$ is a hydrogen atom, an alkyl group having 1 to 6 carbons, $-CH_2-COOH$, or an amino group ($-NH_2$). As the alkyl group having 1 to 6 carbons, groups similar to the groups listed for $R^{11}$ can be used.

In general formula (1-2) and general formula (2-2), $R^{22}$ is a hydrogen atom, an alkyl group having 1 to 6 carbons, or an amino group. As the alkyl group having 1 to 6 carbons, groups similar to the groups listed for $R^{11}$ can be used.

In general formula (1-3) and general formula (2-3), $R^{23}$ is a hydrogen atom or an alkyl group having 1 to 6 carbons. As the alkyl group having 1 to 6 carbons, groups similar to the groups listed for $R^{11}$ can be used.

In general formula (1-4) and general formula (2-4), $R^{24}$ is an alkyl group having 1 to 6 carbons. As the alkyl group having 1 to 6 carbons, groups similar to the groups listed for $R^{11}$ can be used.

In general formula (1-5) and general formula (2-5), $R^{25}$ is an alkyl group having 1 to 6 carbons. As the alkyl group having 1 to 6 carbons, groups similar to the groups listed for $R^{11}$ can be used.

In general formula (1-6) and general formula (2-6), $R^{26}$ is a hydrogen atom or an alkyl group having 1 to 6 carbons. As the alkyl group having 1 to 6 carbons, groups similar to the groups listed for $R^{11}$ can be used.

In general formula (1-7) and general formula (2-7), $R^{27}$ is a hydrogen atom or an alkyl group having 1 to 6 carbons. As the alkyl group having 1 to 6 carbons, groups similar to the groups listed for $R^{11}$ can be used.

In general formula (1-8) and general formula (2-8), $R^{28}$ is a hydrogen atom, an alkyl group having 1 to 6 carbons,

or a cyano group (-CN). As the alkyl group having 1 to 6 carbons, groups similar to the groups listed for $R^{11}$ can be used.

In general formula (1-9) and general formula (2-9), $R^{29}$ is an alkyl group having 1 to 6 carbons. As the alkyl group having 1 to 6 carbons, groups similar to the groups listed for $R^{11}$ can be used.

In general formula (1-10) and general formula (2-10), $R^{30}$ is an alkyl group having 1 to 6 carbons. As the alkyl group having 1 to 6 carbons, groups similar to the groups listed for $R^{11}$ can be used.

In general formula (1-11) and general formula (2-11), $R^{31}$ is a hydrogen atom or an alkyl group having 1 to 6 carbons. As the alkyl group having 1 to 6 carbons, groups similar to the groups listed for $R^{11}$ can be used.

In general formula (1-12) and general formula (2-12), $R^{32}$ and $R^{33}$ are each independently a hydrogen atom or an alkyl group having 1 to 6 carbons. As the alkyl group having 1 to 6 carbons, groups similar to the groups listed for $R^{11}$ can be used. Preferably, $R^{32}$ and $R^{33}$ are each independently a hydrogen atom, a methyl group, or an ethyl group, and more preferably, $R^{32}$ and $R^{33}$ are both a hydrogen atom, a methyl group, or an ethyl group. Moreover, it is also preferable for $R^{32}$ to be a hydrogen atom and $R^{33}$ to be a methyl group or an ethyl group.

In general formula (1-13) and general formula (2-13), $R^{34}$ is a hydrogen atom, an alkyl group having 1 to 6 carbons, $-CH_2-N_3$, a cyano group, an amino group, a nitro group ($-NO_2$), or an azide group ($-N_3$). As the alkyl group having 1 to 6 carbons, groups similar to the groups listed for $R^{11}$ can be used.

In general formula (1-14) and general formula (2-14), $R^{35}$ is an alkyl group having 1 to 6 carbons. As the alkyl group having 1 to 6 carbons, groups similar to the groups listed for $R^{11}$ can be used. $R^{35}$, which occurs twice in one molecule, may be mutually identical groups or different groups. As $R^{35}$, a hydrogen atom, a methyl group, or an ethyl group is preferable; a methyl group or an ethyl group is more preferable; and a methyl group is further preferable.

In general formula (1-15) and general formula (2-15), A is a five- or six-membered heterocyclic group including a nitrogen atom. The nitrogen atom in this heterocyclic group is joined to a carbon atom of a purine ring via a methylene group. As this heterocyclic group, a monovalent group wherein a hydrogen atom that bonds to a nitrogen atom is removed from pyrrolidine, piperidine, morpholine, or thiomorpholine can be mentioned.

As compound (1), the compound represented by general formula (1-13), the compound represented by general formula (1-1), or the compound represented by general formula (1-11) is preferable, and a compound wherein $R^{34}$ in general formula (1-13) is a hydrogen atom, an alkyl group having 1 to 3 carbons, a cyano group, or a nitro group; a compound wherein $R^{21}$ in general formula (1-1) is a hydrogen atom or an alkyl group having 1 to 3 carbons; or a compound wherein $R^{31}$ in general formula (1-11) is a hydrogen atom or an alkyl group having 1 to 3 carbons is more preferable.

As compound (2), the compound represented by general formula (2-13), the compound represented by general formula (2-1), or the compound represented by general formula (2-11) is preferable, and a compound wherein $R^{34}$ in general formula (2-13) is a hydrogen atom, an alkyl group having 1 to 3 carbons, a cyano group, or a nitro group; a compound wherein $R^{21}$ in general formula (2-1) is a hydrogen atom or an alkyl group having 1 to 3 carbons; or a compound wherein $R^{31}$ in general formula (2-11) is a hydrogen atom or an alkyl group having 1 to 3 carbons is more preferable.

It is particularly preferable for the antiviral agent of the present invention to be one or more compounds selected from a group consisting of HUP1136 (tubercidin) (CAS No.: 69-33-0), HUP1108 (5-hydroxymethyltubercidin), HUP1069 (5-formyltubercidin oxime), HUP1077 (tubercidin N-oxide), HUP1078 (5-nitrotubercidin), and HUP1109 (5-cyanotubercidin, toyocamycin).

[Chem. 16]

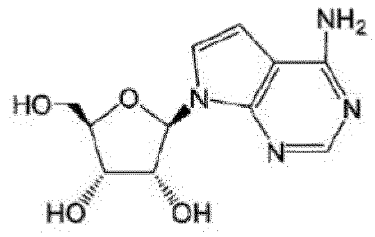

HUP1136

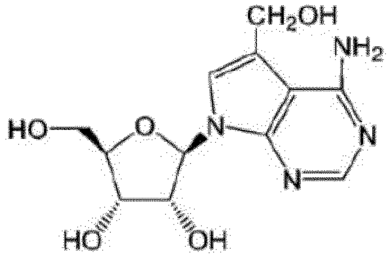

HUP1108

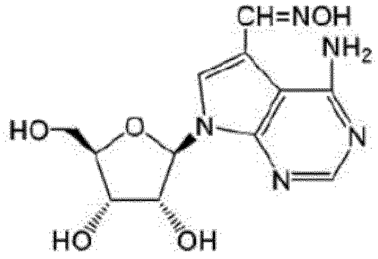

HUP1069

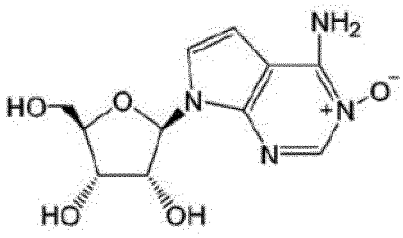

HUP1077

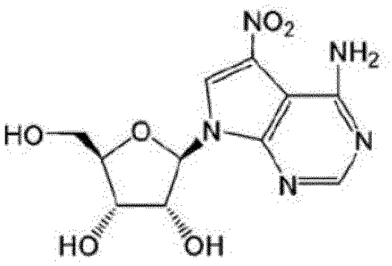

HUP1078

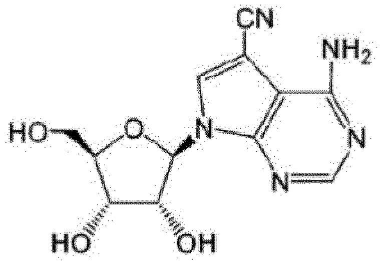

HUP1109

HUP1108, HUP1069, HUP1077, HUP1078, and HUP1109 can be synthesized from HUP1136 by using the method taught in non-patent literature 2. HUP1108 can also be synthesized from HUP1136 by using the method taught in non-patent literature 3 and from sangivamycin, a natural product, by using the method taught in non-patent literature 4.

In the antiviral agent of the present invention, compound (1) and compound (2) may form a salt, and as an acid or a base forming this salt, a mineral acid such as hydrochloric acid or sulfuric acid; an organic acid such as acetic acid, succinic acid, or citric acid; an alkali metal such as sodium or potassium; an alkaline earth metal such as calcium or magnesium; and the like can be mentioned. Moreover, compound (1) and compound (2) may also be in the form of a solvate such as a hydrate.

The antiviral agent of the present invention may be a derivative of compound (1) or a derivative of compound (2). As the derivative of compound (1) or the derivative of compound (2), a derivative that produces compound (1) or compound (2) by undergoing an *in vivo* enzyme treatment or the like is preferable. In the present invention, as the derivative of compound (1) or the derivative of compound (2) that can be used as the antiviral agent, for example, a derivative obtained by prodrugging a pharmaceutical whose active ingredient is a nucleic-acid compound is more preferable. Specifically, a phosphate derivative wherein, in general formula (1) or general formula (2), a hydroxy group joined to a furan ring via a methylene group is substituted by a phosphate group ($PO_4^-$); a monophosphoramidite derivative wherein this hydroxy group is substituted by a monophosphoramidite group; a monophosphorothioate derivative wherein this hydroxy group is substituted by a monophosphorothioate group; and the like can be mentioned. Moreover, the derivative of compound (1) and the derivative of compound (2) may form a salt and be in the form of a solvate such as a hydrate. Those listed above can be used as the salt.

Compound (1), the derivative of compound (1), compound (2), the derivative of compound (2), the salt of these compounds, or the solvate thereof (also collectively referred to as the "nucleic-acid compounds of the present invention" hereinbelow) has antiviral activity against viruses-in particular, against coronaviruses and flaviviruses (flaviviruses)-and is thus useful as an active ingredient of a pharmaceutical composition used to treat or prevent a viral infection.

Examples of coronaviruses include SARS-CoV, MERS-CoV, and SARS-CoV-2. There are various mutant strains of SARS-CoV-2; for example, there are $\alpha$ strain (alpha strain) (B.1.1.7 line), $\beta$ strain (beta strain) (B.1.351 line), $\gamma$ strain (gamma strain) (P.1 line), (delta strain) (B.1.617.2 line), ο strain (omicron strain) (B.1.1.529), E484K strain (R.1 line), (epsilon strain) (B.1.427 / B.1.429 line), $\theta$ strain (theta strain) (P.3 line), $\kappa$ strain(kappa strain) (B.1.617.1 line), and other mutant strains.

Like coronaviruses, flaviviruses are enveloped viruses having positive-strand single-strand RNA as genes. As flaviviruses, the dengue virus (DENV), which causes dengue fever (dengue hemorrhagic fever); the Zika virus (ZIKV), which causes Zika fever (Zika virus disease); the yellow fever virus (YFV), which causes yellow fever; the West Nile virus (WNV), which causes West Nile fever (West Nile encephalitis); and the Japanese encephalitis virus (JEV), which causes Japanese encephalitis, can be mentioned. There are four types of DENV: type 1 to type 4 (DENV1, DENV2, DENV3, DENV4).

The nucleic-acid compounds of the present invention inhibit RNA-dependent RNA polymerase (RdRp), similarly

to remdesivir. Therefore, it has antiviral activity against viruses replicated by RdRp. Examples of such viruses include viruses that cause viral hepatitis, such as coronaviruses, flaviviruses, and *Hepacivirus C.* The nucleic-acid compounds of the present invention can be used for the treatment of infections caused by viruses replicated by RdRp.

The nucleic-acid compounds of the present invention are very useful as an active ingredient of a pharmaceutical composition used to treat or prevent, in particular, COVID-19 and other coronavirus infections. Since all of the various variants of SARS-CoV-2 require RdRp for replication, the nucleic-acid compounds of the present invention have antiviral activity against all of the variants.

Moreover, the nucleic-acid compounds of the present invention are also very useful as an active ingredient of a pharmaceutical composition used to treat or prevent dengue fever, Zika fever, yellow fever, West Nile fever, Japanese encephalitis, and other flavivirus infections. The nucleic-acid compounds of the present invention are low-molecular-weight compounds and thus have no problems in terms of immunogenicity and the like. Moreover, because they can also be administered orally and are not very limited in terms of administration route, they are particularly useful as an active ingredient of a drug for mammals, including humans.

Non-patent literature 7 examines the incorporation of GS-441524 into cells. The conjecture of the authors is that the incorporation of GS-441524 into cells is reduced because the incorporation of GS-441524 into cells is completely dependent on adenosine transporters present on the cell surface and these transporters are reduced in hypoxic pulmonary epithelial cells and certain cell strains.

One major difference between GS-441524 and tubercidin derivatives such as HTMU is that a cyano group (-CN) is bonded to the first position of GS-441524 but not bonded to HTMU. Therefore, it is possible that this difference is one reason why the compounds of the present invention exhibit greater efficacy compared to GS-441524.

Therefore, the compounds of the present invention are believed to be incorporated into cells via a plurality of mechanisms. This is thought to improve the problem of reduced incorporation into hypoxic pulmonary epithelial cells having reduced adenosine transporters and provide excellent antiviral activity.

When one type or two or more types among the nucleic-acid compounds of the present invention are contained in a pharmaceutical composition, as necessary, these can be combined with a pharmaceutically acceptable carrier, and various administration forms can be adopted according to the preventive or therapeutic object. As these forms, for example, an oral agent, an injection, a suppository, an ointment, and a patch can be mentioned, although an oral agent is preferable. These administration forms can be respectively produced by formulation methods well known to persons skilled in the art.

As the pharmaceutically acceptable carrier, an excipient, a binder, a disintegrant, a lubricant, or a colorant in a solid preparation; a solvent, a solubilizer, a suspending agent, a tonicity agent, a buffer, or an analgesic in a liquid preparation; or the like is used. Moreover, as necessary, a preparation additive such as an antiseptic, an antioxidant, a colorant, a sweetener, or a stabilizer can also be used.

When preparing an oral solid preparation, an excipient and, as necessary, a binder, a disintegrant, a lubricant, a colorant, a flavor/odor corrigent, or the like is added to the nucleic-acid compounds of the present invention. Afterward, a tablet, a coated tablet, granules, a powder, a capsule, or the like can be produced by a conventional method.

When preparing an oral liquid preparation, a flavor corrigent, a buffer, a stabilizer, an odor corrigent, and the like are added to the nucleic-acid compounds of the present invention, and an internal liquid agent, a syrup, an elixir, or the like can be produced by a conventional method.

When preparing an injection, a pH regulator, a buffer, a stabilizer, a tonicity agent, a local anesthetic, and the like are added to the nucleic-acid compounds of the present invention, and hypodermic, intramuscular, and intravenous injections can be produced by conventional methods.

When preparing a suppository, a preparation carrier that is well known in the field-for example, polyethylene glycol, lanolin, cacao butter, or a fatty-acid triglyceride-is added to the nucleic-acid compounds of the present invention. Afterward, the suppository can be produced by a conventional method.

When preparing an ointment, a base, a stabilizer, a humectant, a preservative, and the like that are normally used are combined as necessary into the nucleic-acid compounds of the present invention, and these are mixed and formulated by a conventional method.

When preparing a patch, it is sufficient to coat the above ointment, a cream, a gel, a paste, or the like onto a normal supporting body by using a conventional method.

A content of the nucleic-acid compounds of the present invention in each of the above preparations depends on patient symptoms, a dosage form of the preparation, and the like and is not constant. However, generally, it is approximately 0.001 to 1,000 mg in an oral agent, approximately 0.001 to 500 mg in an injection, and approximately 0.01 to 1,000 mg in a suppository.

Furthermore, an administration amount per day of these preparations differs according to patient symptoms, bodyweight, age, sex, and the like, and no sweeping determination can be made for such. However, it is approximately 0.005 to 5,000 mg per day for a normal adult (60 kg bodyweight). 0.01 to 1,000 mg is preferable, and administering this once a day or divided over about two to three times a day is preferable.

Moreover, the dose of the nucleic-acid compounds of the present invention is not particularly limited, but it is preferably less than 60 mg/kg, more preferably 10 mg/kg or more and less than 60 mg/kg, and even more preferably 15 to 40 mg/kg.

For example, the dose of the nucleic-acid compounds of the present invention may be 15 to 30 mg/kg, and it may also be 20 mg/kg. Further, for example, HUP1108 (5-hydroxymethyltubersidin) of less than 60 mg/kg may be administered, and administering 20 mg/kg HUP1108 is preferable.

An animal whereto an antiviral agent and pharmaceutical composition whose active ingredient is the nucleic-acid compounds of the present invention is administered is not particularly limited and may be a human or an animal other than a human. As the nonhuman animal, a mammal such as a cow, a pig, a horse, a sheep, a goat, a monkey, a dog, a cat, a rabbit, a mouse, a rat, a hamster, or a guinea pig; a bird such as a chicken, a quail, or a duck; and the like can be mentioned.

[Examples]

Next, the present invention is described in greater detail by giving examples. However, the present invention is not limited to the following examples.

<Coronaviruses>

Among the coronaviruses used in the following tests, as SARS-CoV-2, strain JPN/TY/WK-521 distributed by the National Institute of Infectious Diseases was used. Moreover, as variant strains of SARS-CoV-2, QK002 (strain alpha), TY8-612 (strain beta), TY5-501 (strain gamma), and TY11-927 (strain delta) distributed by the National Institute of Infectious Diseases were used.

HCoV-229E (ATCC: VR-740) and HCoV-OC43 (ATCC: VR-1558) obtained from the ATCC were used as coronaviruses that routinely infect humans (human coronaviruses: HCoV).

<Flaviviruses>

Among the flaviviruses used in the following tests, strains D1/hu/PHL/10-07, D2/hu/INDIA/09-74, D3/hu/Thailand/00-40, and D4/hu/Solomon/08-11 were respectively used as DENV1-4. Strain MR766 was used as ZIKV. Strain 17D-204 was used as YFV. Strain NY99 was used as WNV. Strain Beijing-1 was used as JEV.

<Culture Cells>

In the following tests, culture-cell strain MRC5 cells from the lungs of a human fetus or culture-cell strain Caco-2 cells from a human colon carcinoma were infected using the viruses. For both cell types, 2% FBS/MEM was used as a culture medium. The 2% FBS/MEM was prepared by adding 2% FBS (fetal bovine serum; made by Gibco) and L-glutamine (made by Wako) to MEM (Minimum Essential Medium; made by Nissui).

[Example 1]

Using compounds from a library, possessed by the Center for Research and Education on Drug Discovery in the Faculty of Pharmaceutical Sciences at Hokkaido University, of compounds having a nucleic-acid compound as a backbone as test samples, screening was performed for compounds exhibiting an effect of suppressing cell death of cells infected by a human coronavirus. HCoV-229E and HCoV-OC43 were used as the human coronavirus.

As a result, it was found that among the compounds included in the compound library that was used, the following six compounds-HUP1136, HUP1108, HUP1069, HUP1077, HUP1078, and HUP1109-had antiviral activity.

[Chem. 17]

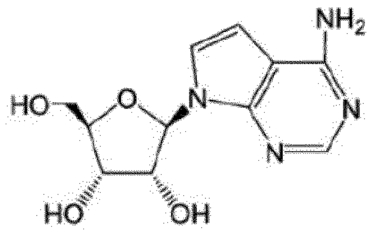

HUP1136

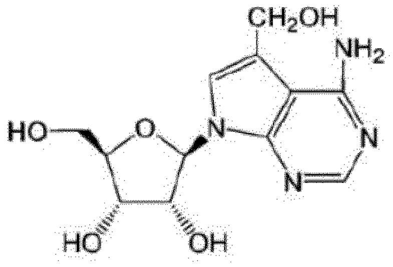

HUP1108

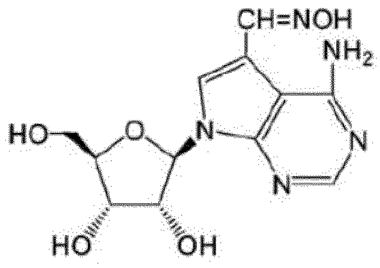

HUP1069

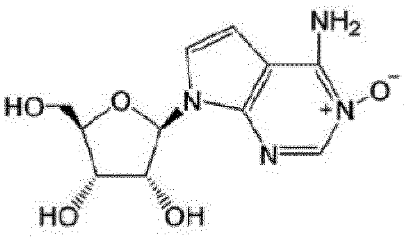

HUP1077

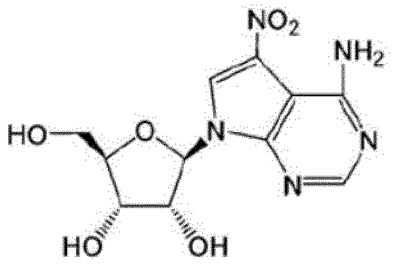

HUP1078

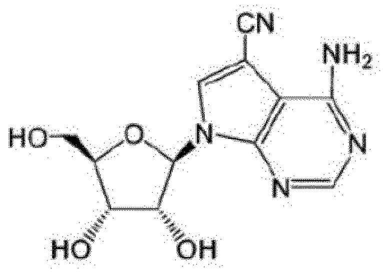

HUP1109

<Confirmation of Cell-Death Suppression Effect>

An effect of suppressing cell death due to a coronavirus infection was examined for the six compounds evaluated as having antiviral activity. As a positive control, GS-5734 (remdesivir) and GS-441524 were examined in the same manner.

First, in each well of a 96-well plate, cells treated by each compound were infected using the viruses, and these were cultured for three days in a $CO_2$ incubator (37°C, 5% $CO_2$).

The 96-well plate cultured for three days was observed macroscopically and microscopically, and a cell form, a presence or absence of crystals, and the like were confirmed. Afterward, 20 μL. of a resazurin solution (a solution wherein resazurin sodium salt (made by Sigma) is dissolved in PBS so that 0.24 mg/mL is achieved) was dispensed into each well. After culturing this for 2 hours in a $CO_2$ incubator, 20 μL of a reaction-stopping solution (solution wherein a virus inactivation solution-sodium dodecyl sulfate (SDS)-is dissolved in distilled water so that 7.5% is achieved) was dispensed into each well, and this was blended using a plate mixer.

Afterward, a fluorescent intensity of this 96-well plate at 531 nm (emission) / 590 nm (excitation) was measured using a multi-plate reader (device name: EnVision; made by PerkinElmer).

Using statistical analysis software (product name: GraphPad Prism 8; made by GraphPad Software), a 50% cell death inhibition concentration ($EC_{50}$) of the virus-infected cells was calculated based on a calculation formula such as the following. Moreover, from values of $EC_{50}$ and $CC_{50}$, SI ($EC_{50}/CC_{50}$) was calculated. Calculation results are given in Table 1.

[Agonist] vs response-Variable slope (four parametric)

$$Y = Bottom + (X^{HillSlope}) * (Top - Bottom) / (X^{HillSlope} + EC50^{HillSlope})$$

X: Concentration of test sample
Y: Infected-cell death inhibition rate (cell survival rate)
HillSlope: Slope of series of curves
Bottom (base reaction) = 0 (infected-cell death inhibition rate: 0%)
Top (maximum reaction) = 100 (infected-cell death inhibition rate: 100%)

[Table 1]

| | HCoV-OC43 | | HCoV-229E | | $CC_{50}$ (μM) |
|---|---|---|---|---|---|
| | $EC_{50}$ (μM) | SI | $EC_{50}$ (μM) | SI | |
| HUP1136 | 0.11 | 5.9 | < 0.078 | > 8. a | 0.66 |
| HUP1108 | 0.35 | > 141 | 0.43 | > 117 | > 50 |
| HUP1069 | 0.055 | 13 | < 0.078 | > 9.5 | 0.74 |
| HUP1077 | 4.3 | 5.6 | 4.0 | 6. 1 | 24 |
| HUP1078 | > 10 | | 2.0 | 0.17 | 0.33 |
| HUP1109 | > 10 | | 2.3 | 0.26 | 0.59 |
| GS-441524 | 3.7 | > 13. 7 | 3.1 | > 16.1 | > 50 |
| GS-5734 | 0.23 | > 221.1 | 0.072 | >694. 1 | > 50 |

[Example 2]

A viral-RNA replication suppression effect against SARS-CoV-2 was examined for the six compounds confirmed to have antiviral activity in example 1. Caco-2 cells were used as culture cells infected by the virus.

<Confirmation of Viral-RNA Replication Suppression Effect>

(1) Viral Infection of Culture Cells

A culture medium made of 2% FBS/MEM was used to dilute the test samples (the six compounds confirmed to have antiviral activity), the virus, and the cells.

First, a Caco-2 cell solution prepared to an appropriate cell count was dispensed at 100 μL/well in a 96-well plate, and this was cultured overnight by a $CO_2$ incubator (37°C, 5% $CO_2$). Next, in each well of this 96-well plate wherein the Caco-2 cells were placed, the test samples, which were diluted in advance to an appropriate concentration using the culture medium, were added so as to produce a triple-stage dilution series (50 μL/well), and these were blended using a plate mixer. Into this 96-well plate containing the test samples, a viral solution diluted in advance to an appropriate concentration using the culture medium was dispensed at 50 μL/well. Afterward, this 96-well plate was blended using a plate mixer and cultured for 48 hours by a $CO_2$ incubator (37°C, 5% $CO_2$).

(2) RNA Extraction and Purification

After the 48-hour culturing, 150 μL each of the culture supernatant was collected from each well of the 96-well plate. Moreover, the virus was inactivated by adding a lysis buffer included in an RNA extraction kit (product name: PureLink Pro 96 Viral RNA/DNA Kit; made by Thermo Fischer Scientific) to each well. Afterward, the viral RNA was purified according to the protocols included in this kit.

(3) Measurement of Viral RNA

qRT-PCR (real-time quantitative reverse transcription PCR) was implemented using the purified viral RNA and a probe/primer set designed in a nucleocapside region of SARS-CoV-2. The qRT-PCR was performed using a commercially available kit (product name: THUNDERBIRD Probe One-Step qRT-PCR Kit; made by TOYOBO) and a real-time PCR system (product name: QuantStudio 7 Flex Real-Time PCR System; made by Thermo Fisher Scientific).

A number of viral-RNA copies in each sample was calculated using a calibration-curve method. The calibration curve was made by performing qRT-PCR in the same manner by using, as a template, a plasmid incorporating a base sequence including the above nucleocapside region ($10^9$ to $10^3$ copies).

(4) Calculation of Viral-RNA Replication Inhibition Rate

Based on a number of viral-RNA copies in a group having no test samples added thereto, a viral-RNA replication inhibition rate (%) of the test samples at each concentration was calculated. FIG. 1 illustrates measurement results of a viral-RNA amount ($\log_{10}$ [number of viral-RNA copies]) from cells treated by each test sample, and FIG. 2 illustrates

measurement results of the viral-RNA replication inhibition rate ([number of viral-RNA copies from cells treated by test samples]/[number of viral-RNA copies from cells of group having no test samples added thereto]×100%).

Afterward, based on a calculation formula such as the following, a 90% viral-RNA replication inhibition concentration ($EC_{90}$) was calculated using statistical analysis software (product name: GraphPad Prism 8; made by GraphPad Software). Calculation results are given in Table 2.

[Agonist] vs response- Find ECanything

$$EC50 = ECF / (F / (100 - F))^{(1 / HillSlope)}$$

$$Y = Bottom + (Top - Bottom) / (1 + (EC50 / X)^{HillSlope})$$

ECF: Concentration of test sample whereat reaction value is F%, between Bottom and Top
F: Value of target inhibition rate (for $EC_{90}$, F = 90)
X: Concentration of test sample
Y: Viral-RNA replication inhibition rate
HillSlope: Slope of series of curves
Bottom (base reaction) = 0 (viral-RNA replication inhibition rate: 0%)
Top (maximum reaction) = 100 (viral-RNA replication inhibition rate: 100%)

[Table 2]

| | SARS-CoV-2 |
|---|---|
| | $EC_{90}$ (μM) |
| HUP1136 | 0.087 |
| HUP1108 | 0.57 |
| HUP1069 | 0.20 |
| HUP1077 | 3.5 |
| HUP1078 | 0.**10** |
| HUP1109 | 0.20 |

As illustrated in FIG. 1 and FIG. 2, for the cells treated by the six compounds confirmed to have antiviral activity in example 1, although varying according to the compound, a tendency was observed wherein, in a manner dependent on an amount of the treatment compound, a virus amount released from the infected cells decreased and a viral-RNA replication inhibition rate increased. This confirmed that these compounds have antiviral activity against SARS-CoV-2.

As a result of microscopic observation during implementation of tests, strong cytotoxicity was observed for the cells treated by HUP1109, to an extent of cell extinction. Moreover, mild cytotoxicity, not to an extent of cell extinction, was observed for HUP1078 (0.123 μM~), HUP1136 (1.11 μM~), and HUP1108 (10 μM). In the cells treated by HUP1078 and HUP 1109, a possibility was suggested that antiviral activity is being exhibited due to the cytotoxic effect of these compounds. Meanwhile, for HUP1136 and HUP1108, the concentrations whereat antiviral activity was confirmed were of a lower concentration range than cytotoxicity. For HUP1108 in particular, in a concentration range higher than 3.33 μM, there is a possibility of virus proliferation being suppressed due to a cytotoxic effect, but virus proliferation was suppressed even in a concentration range lower than 3.33 μM, and antiviral activity was confirmed even in a low-concentration region exhibiting no cytotoxicity. Among these six compounds, concerns regarding cytotoxicity were low for HUP1077 and HUP1108 in particular. These compounds exhibited concentration-dependent viral-RNA replication inhibition activity and were thought to be very useful as an antiviral agent.

[Example 3]

A time-of-addition test was performed to examine a point of action of anti-SARS-CoV-2 activity of HUP1108. Caco-2 cells were used as culture cells infected by the virus.

<Time-of-Addition Test>

(1) Viral Infection of Culture Cells

A culture medium made of 2% FBS/MEM was used to dilute the test sample (HUP1108), the virus, and the cells.

First, 300 μL/well of a Caco-2 cell solution prepared to an appropriate cell count was dispensed into a 48-well plate, and this was cultured overnight by a $CO_2$ incubator (37°C, 5% $CO_2$). Next, the culture supernatant was removed from each well containing the Caco-2 cells in this 48-well plate, and afterward, a viral solution diluted in advance to an appropriate concentration using the culture medium was added (150 μL/well). Moreover, the test sample, diluted in advance using the culture medium, was added at the following three timings (Whole, Entry, and Post-Entry) (150 μL/well; final concentration: 1 μM). After blending this using a plate mixer, this was cultured for a total of 24 hours by a $CO_2$ incubator (37°C, 5% $CO_2$).

• Whole: Test sample present in all steps of viral infection

The test sample was added at the same timing as adding the viral solution, and this was blended using a plate mixer. Afterward, this was cultured for 1 hour by a $CO_2$ incubator (37°C, 5% $CO_2$). Afterward, the culture supernatant was removed. After washing using the culture medium, new culture medium containing the test sample was added, and this was cultured for 24 hours by a $CO_2$ incubator (37°C, 5% $CO_2$).

• Entry: Test sample present only at time of virus entry

The test sample was added at the same timing as adding the viral solution, and this was blended using a plate mixer. Afterward, this was cultured for 1 hour by a $CO_2$ incubator (37°C, 5% $CO_2$). Afterward, the culture supernatant was removed. After washing using the culture medium, new culture medium containing the test sample was added, and this was cultured for 2 hours by a $CO_2$ incubator (37°C, 5% $CO_2$). Afterward, the culture supernatant was again removed. After washing using the culture medium, the culture medium-including no test sample-was added, and this was cultured for 22 hours by a $CO_2$ incubator (37°C, 5% $CO_2$).

• Post-Entry: Test sample present only after virus entry

No test sample was added when adding the virus, and this was cultured for 1 hour by a $CO_2$ incubator (37°C, 5% $CO_2$). Afterward, the culture supernatant was removed. After washing using the culture medium, new culture medium (culture medium containing no test sample) was added, and this was cultured for 2 hours by a $CO_2$ incubator (37°C, 5% $CO_2$). Afterward, the culture supernatant was again removed. After washing using the culture medium, the culture medium-containing the test sample-was added, and this was cultured for 22 hours by a $CO_2$ incubator (37°C, 5% $CO_2$).

(2) RNA Extraction and Purification

After culturing for 24 hours, the culture supernatant was collected from each well of the 48-well plate and used for virus titration. The cells in the wells were washed using PBS (phosphate-buffered saline) (300 μL × twice). Moreover, a lysis buffer included in an RNA extraction kit (product name: PureLink RNA Mini Kit; made by Thermo Fisher Scientific) was added to each well to dissolve the cells. Afterward, total RNA was purified according to the protocols included in this kit.

(3) Measurement of Viral RNA

qRT-PCR was implemented using the purified total RNA and a probe/primer set designed in a nucleocapside region of SARS-CoV-2. The qRT-PCR was performed in the same manner as example 2 other than using ACTB as an endogenous control.

(4) Calculation of Viral-RNA Replication Inhibition Rate

Based on a Ct value of each sample obtained by the qRT-PCR, the ΔΔ Ct method was used to calculate a relative viral-RNA amount in a situation wherein a viral-RNA amount of a group having no test sample added thereto is defined as 1, and a viral-RNA replication inhibition rate was calculated. Calculation results of the relative viral-RNA amount of each sample are given in FIG. 3. The graph illustrated in FIG. 3 was made using statistical analysis software (product name: GraphPad Prism 8; made by GraphPad Software).

(5) Virus Titration

A solution wherein the culture supernatant collected at (2) above is diluted to an appropriate concentration using the culture medium was added (100 μL/well) to VeroE6/TMPRSS2 cells dispensed into a 96-well plate (100 μL/well), and this was blended using a plate mixer. Afterward, this was cultured for 72 hours by a $CO_2$ incubator (37°C, 5% $CO_2$). Afterward, a cytopathic effect (CPE) due to the viral infection was observed, and a median tissue culture infectious dose ($TCID_{50}$) was calculated. Logarithm values of the calculated $TCID_{50}$ are given in FIG. 4. The graph illustrated in FIG. 4 was made using statistical analysis software (product name: GraphPad Prism 8; made by GraphPad Software).

As illustrated in FIG. 3 and FIG. 4, a reduced relative viral-RNA amount and viral titer were observed when an HUP1108 treatment timing was Whole and Post-Entry. These results suggest that the point of action of anti-SARS-CoV-2 activity of HUP1108 is during the replication process after virus entry.

[Example 4]

An effect of suppressing cell death due to a flavivirus infection was examined for the six compounds evaluated as having antiviral activity in example 1.

<Evaluation of Cell-Death Suppression Effect>

(1) Culture Cells

In the following tests, culture-cell strain BHK-21 cells from Syrian hamster kidneys were infected using the viruses. 2% FBS/MEM or 2% FBS/RPMI-1640 was used as a culture medium. Each culture medium was prepared by adding 2% FBS (fetal bovine serum; made by Gibco) and L-glutamine (made by Wako) to MEM (minimum essential medium; made by Nissui) or RPMI-1640 (made by Gibco).

(2) Viral Infection and MTT Assay

First, cells treated by each compound were infected by each type of virus in each well of a 96-well plate. DENV2 was cultured for four days; DENV1, 3, and 4 were cultured for five days; and ZIKV, YFV, WNV, and JEV were cultured for three days in a $CO_2$ incubator (37°C, 5% $CO_2$).

The cultured 96-well plate was observed macroscopically and microscopically, and a cell form, a presence or absence of crystals, and the like were confirmed. Next, 30 μL of an MTT solution (solution wherein 3-(4,5-dimethyl-2-thiazol)-2,5-diphenyl-2H-tetrazolium bromide (made by Nacalai Tesque) is dissolved in PBS so that 5 μg/mL is achieved) was added to each well, and this was cultured for 2 hours in a $CO_2$ incubator. Afterward, the supernatant was removed 150 μL at a time from this plate so as to not suction the cells, a cell solution (virus inactivation solution: solution prepared by adding 50 mL of Triton X-100 and 4 mL of hydrochloric acid (12 N) into 500 mL of isopropanol) was added to each well 150 μL at a time, and this was blended using a plate mixer. Afterward, an absorbance of two wavelengths-570 nm and 630 nm-of this 96-well plate was measured using an absorption spectrometer.

(3) Calculation of $EC_{50}$

Based on the following calculation formula, Microsoft Excel or a program having an equivalent calculation processing performance was used to calculate a 50% cell death inhibition concentration ($EC_{50}$) of the virus-infected cells. Results are given in Table 3.

$$[EC_{50}] = 10^{Z}$$

$$Z = ([50\%\ OD] - [Low\ OD]) / ([High\ OD] - [Low\ OD]) \times \{\log(High\ conc.) - \log(Low\ conc.)\} + \log(Low\ conc.)$$

$$[50\%\ OD] = \{OD\ (cell\ control) - OD\ (virus\ control)\} \times 0.5 + OD\ (virus\ control)$$

OD: Absorbance
conc.: drug concentration
OD (cell control): average value of absorbance of wells of control
OD (virus control): average value of absorbance of wells of virus control

$EC_{50}$ was calculated from the absorbance and two points interposing a 50% OD value on a drug concentration curve: A-High (high OD, high conc.) and B-Low (low OD, low conc.).

The results are given in Table 3. As indicated in Table 3, it was found that among the six compounds, the four compounds HUP1136, HUP1108, HUP1069, and HUP1077 all had high antiviral activity against DENV1, DENV2, DENV3, DENV4, ZIKV, YFV, WNV, and JEV.

Furthermore, non-patent literature 5 reports that the antiviral activity ($EC_{50}$) of GS-441524 against DENV2 is 9.46 $\mu$M. This value is remarkably higher than any among compounds HUP1136, HUP1108, HUP1069, and HUP1077 of the present invention. In particular, it is nearly ten times higher than HUP1108 and nearly 40 times higher than HUP1136.

Furthermore, non-patent literature 5 reports that the antiviral activity ($EC_{50}$) of GS-441524 against YFV is 11 $\mu$M. This value is remarkably higher than both HUP1136 and HUP1108. In particular, it is no less than approximately 40 times higher than HUP1108 and no less than approximately 73 times higher than HUP1136.

Therefore, it is clear that the antiviral activities of the compounds of the present invention are remarkably higher than GS-441524.

[Table 3]

| | $EC_{50}$ ($\mu$M) | | | | | | | |
|---|---|---|---|---|---|---|---|---|
| | DENV1 | DENV2 | DENV3 | DENV4 | JEV | ZIKV | YFV | WNV |
| HUP1136 | >16 | <0. 125 | <0.125 | >16 | <0. 13 | | 0.15 | 0.2 |
| HUP1108 | 0.35 | 0.24 | 0.42 | 0.52 | 0.5 | 1.3 | 0.27 | 0.95 |
| HUP1069 | >16 | <0.125 | >16 | >16 | <0. 13 | | | <0. 13 |
| HUP1077 | 3.2 | 0.95 | 2.3 | 2.5 | 6.9 | 6.3 | | 10. 7 |
| HUP1078 | | >20 | | | | | | |
| HUP1109 | | >20 | | | | | | |

[Example 5]

A viral-RNA replication suppression effect of HUP1108 against SARS-CoV-2 (strain WK-521) and various variants thereof (strain $\alpha$, strain $\beta$, strain $\gamma$, strain $\delta$) was examined by a method similar to example 2. However, when infecting the culture cells using the virus, test samples diluted in advance to an appropriate concentration using the culture medium were added (50 $\mu$L/well) to each well of a 96-well plate-each of these wells containing Caco-2 cells-so as to produce a double-stage dilution series instead of a triple-stage dilution series. This was blended using a plate mixer.

As illustrated in FIG. 5, it was observed that a virus amount released from infected cells tended to decrease in a manner dependent on an amount of the HUP1108 compound, confirming that HUP1108 has antiviral activity against various variant strains of SARS-CoV-2.

[Example 6]

*(1) In Vivo* Mice Model (Intrapulmonary Virus-Amount Measurement Model)

BALB/c mice were transnasally inoculated using SARS-CoV-2 (strain WK-521), and the next mice were inoculated using the pulmonary homogenate supernatant from three days following infection. This subculturing was repeated ten times. Beginning at passage 3 (P3), high viral titers were consistently obtained, and from P9 onward, some individuals began to die. A genetic analysis of the P10 virus was implemented by next-generation sequencing (NGS). As a result, a Q498H mutation was observed in the spike-protein region determining the infectivity of the virus.

An effect of HUP1108 in suppressing intrapulmonary viral proliferation in mice was examined by a proliferation evaluation model using this virus that had adapted to the mice (strain MA-P10). Five-week-old BALB/c mice were transnasally inoculated using strain MA-P10 ($2\times10^2$ TCID50/mouse). The day following infection, the lungs of each

individual were collected, and the intrapulmonary viral titers were measured (five mice per group). As a result, an intrapulmonary virus amount in a group that had been intramuscularly administered with HUP1108 2 hours prior to infection (single dose, 20 mg/kg) was approximately 1.3 log lower than an intrapulmonary virus amount in a group administered with only a medium including no drug (vehicle) (FIG. 6).

*(2) In Vivo* Mice Model (Fatality Model)

Next, a fatality suppression effect of HUP1108 was examined using this mice model infected by strain MA-P10.

30- to 50-week-old BALB/c mice were transnasally inoculated using SARS-CoV-2 (strain MA-P10; $2\times10^2$ TCID50 / mouse). HUP1108 was intramuscularly administered (20 mg/kg) one time 2 hours prior to infection and once a day from the day following infection until five days following infection for a total of five days, and the survival rate was calculated (five mice per group). As a result, a survival rate of a group of mice administered with HUP1108 was 100% whereas a survival rate of a group of mice administered for five days with only a medium including no drug (vehicle) was 40% (FIG. 7). That is, consecutive administration of HUP1108 provided a result of suppressing fatal effects of SARS-CoV-2.

Non-patent literature 6 reports that orally administering GS-621763 at no less than 60 mg/kg per day or MPV (molnupiravir) at no less than 120 mg/kg per day to a SARS-CoV-2-MA10 model improves acute respiratory distress syndrome (acute respiratory distress syndrome).

In contrast, intramuscularly administering compound HUP1108 of the present invention at 20 mg/kg per day to the same mice model dramatically reduced an intrapulmonary virus amount, and a survival rate was 100%.

Note that while a direct comparison may not be possible between example 6 and non-patent literature 6 due to the different administration methods, this confirms that even *in vivo,* HUP1108 is a compound exhibiting very high antiviral activity against SARS-CoV-2 in low-dosage use.

## Claims

1. An antiviral agent, comprising: the compound represented by general formula (1) or (2) below-

[Chem. 1]

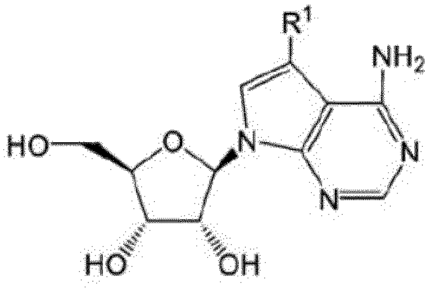

(1)

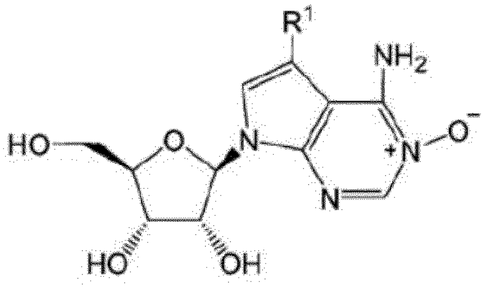

(2)

[in the formulas, $R^1$ being $-(CH_2)n_1-Z^1-R^{11}$ or $-CH-(-Z^1-R^{11})_2$ ($n_1$ being 0 or 1; $Z^1$ being a single bond, an oxygen atom, -NH-, a sulfur atom, -SO-, $-SO_2-$, -CO-, -CO-O-, or -CH=NO-; $R^{11}$ being a hydrogen atom, an alkyl group having 1 to 6 carbons, $-NR^{12}R^{13}$, $-N_3$, - $NO_2$, -CN, $-CH_2-CO-O-R^{14}$, or a five- or six-membered heterocyclic group including a nitrogen atom; $R^{12}$ and $R^{13}$ each independently being a hydrogen atom or an alkyl group having 1 to 6 carbons; and $R^{14}$ being a hydrogen atom or an alkyl group having 1 to 6 carbons-however, the nitrogen atom in the heterocyclic group and $Z^1$ being joined)]-a derivative thereof, a salt of these compounds, or a solvate thereof.

2. The antiviral agent of claim 1, wherein general formula (1) is any among general formulas (1-1) to (1-15) below

[Chem. 2]

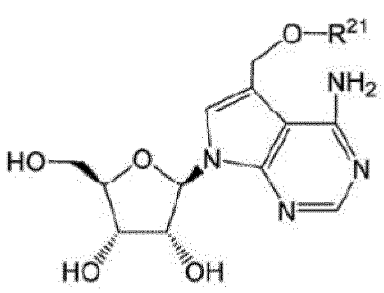

(1-1)

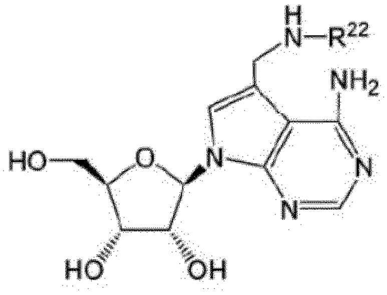

(1-2)

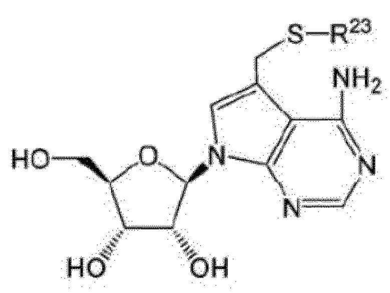

(1-3)

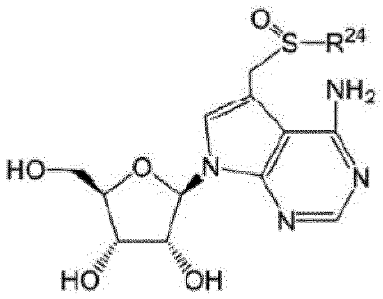

(1-4)

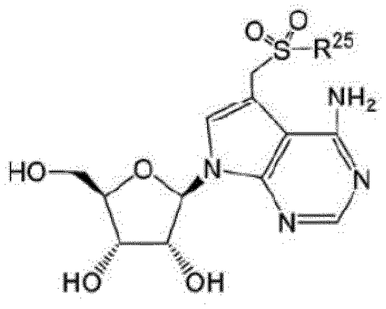

(1-5)

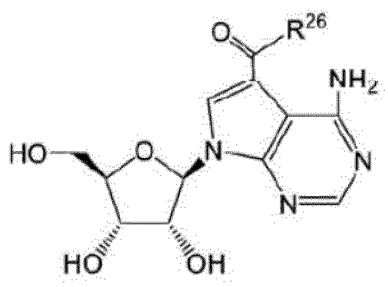

(1-6)

[Chem. 3]

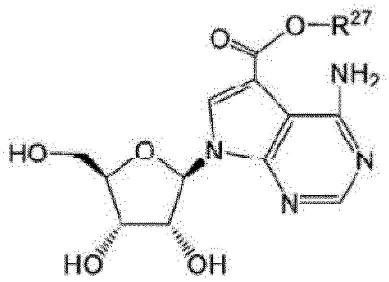

(1-7)

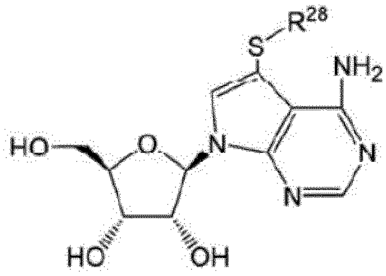

(1-8)

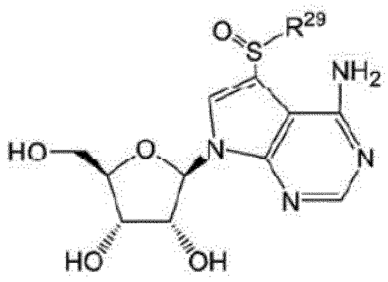

(1-9)

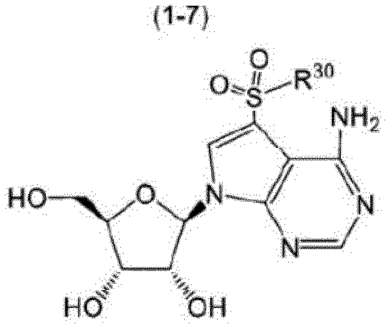

(1-10)

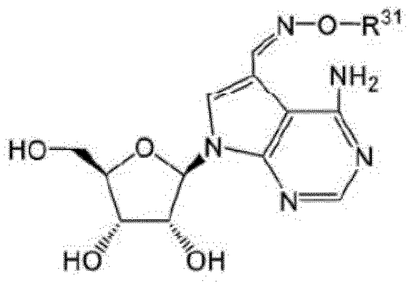

(1-11)

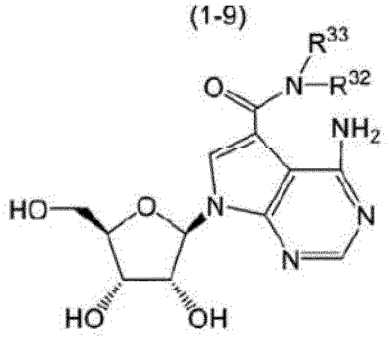

(1-12)

[Chem. 4]

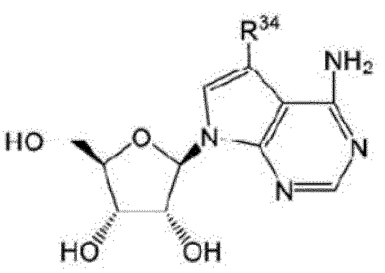

(1-13)

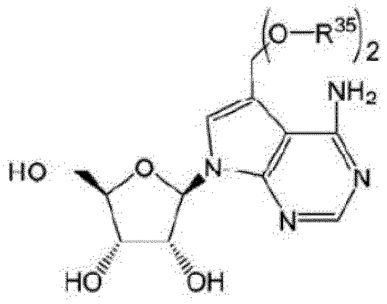

(1-14)

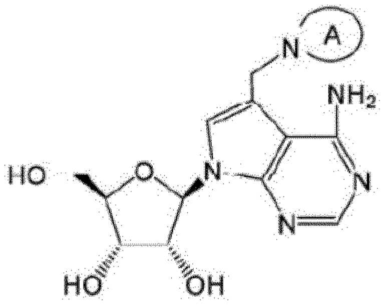

(1-15)

[in the formulas, $R^{21}$ being a hydrogen atom, an alkyl group having 1 to 6 carbons, $-CH_2-COOH$, or an amino group; $R^{22}$ being a hydrogen atom, an alkyl group having 1 to 6 carbons, or an amino group; $R^{23}$, $R^{26}$, $R^{27}$, $R^{31}$, $R^{32}$, and $R^{33}$ being a hydrogen atom or an alkyl group having 1 to 6 carbons; $R^{24}$, $R^{25}$, $R^{29}$, $R^{30}$, and $R^{35}$ being an alkyl group having 1 to 6 carbons; $R^{28}$ being a hydrogen atom, an alkyl group having 1 to 6 carbons, or a cyano group; $R^{34}$ being a hydrogen atom, an alkyl group having 1 to 6 carbons, $-CH_2-N_3$, a cyano group, an amino group, a nitro group, or an azide group; and A being a five- or six-membered heterocyclic group including a nitrogen atom]
and general formula (2) is any among general formulas (2-1) to (2-15) below

[Chem. 5]

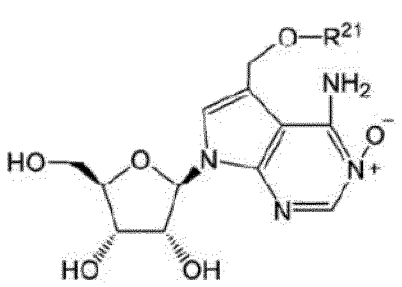

(2-1)

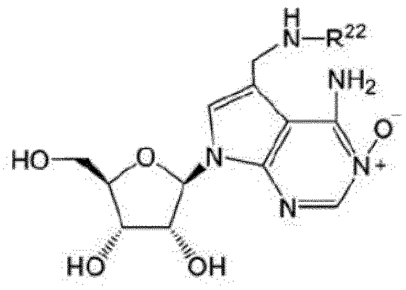

(2-2)

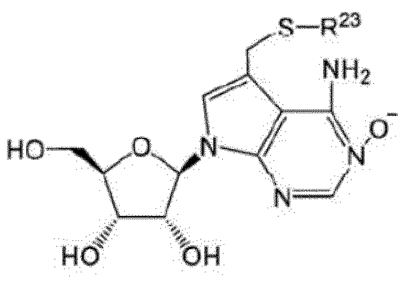

(2-3)

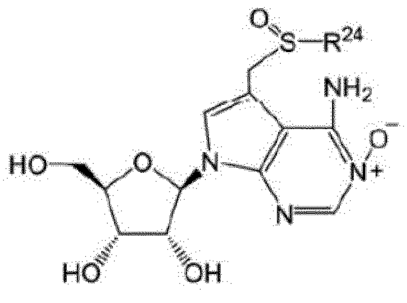

(2-4)

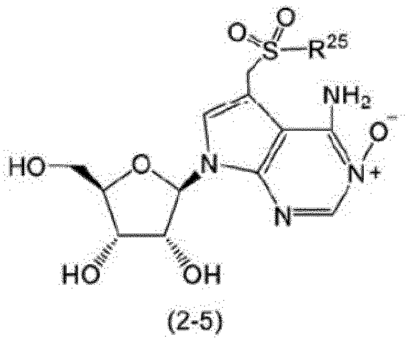

(2-5)

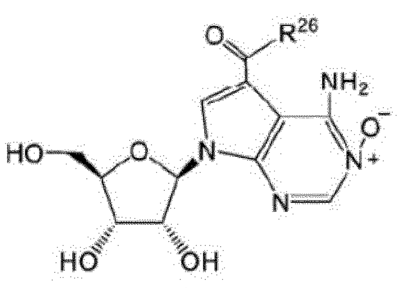

(2-6)

[Chem. 6]

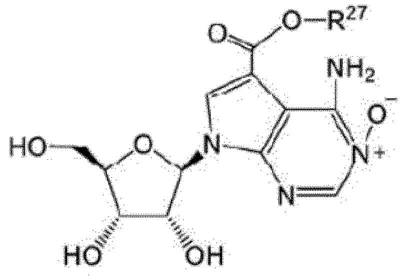
(2-7)

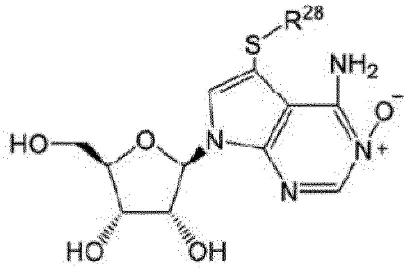
(2-8)

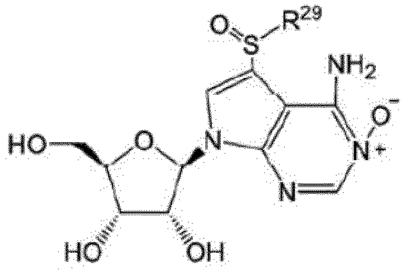
(2-9)

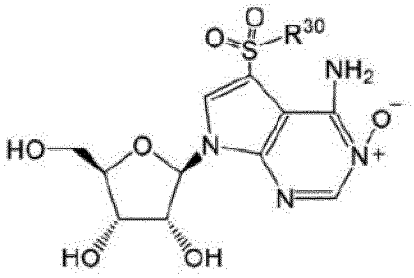
(2-10)

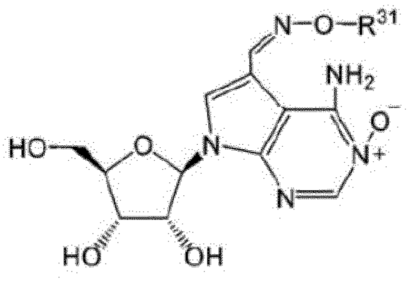
(2-11)

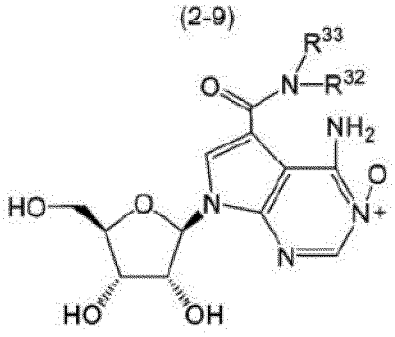
(2-12)

[Chem. 7]

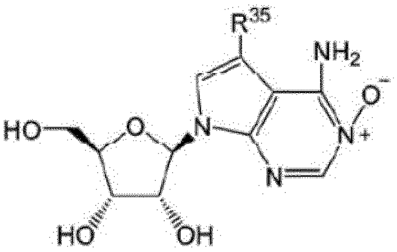
(2-13)

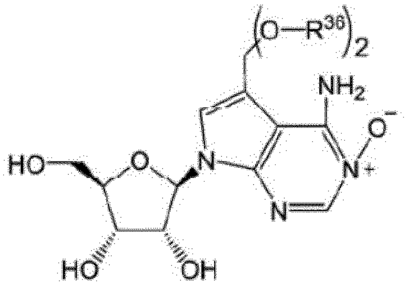
(2-14)

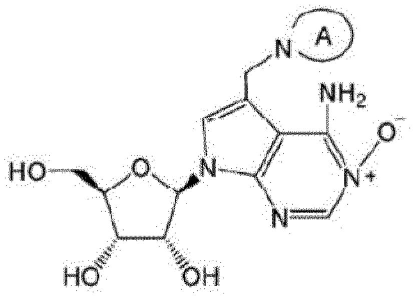
(2-15)

[in the formulas, $R^{21}$ to $R^{35}$ and A being as above].

**3.** The antiviral agent of claim 1 or 2, wherein the antiviral agent is made of one or more compounds selected from a group consisting of HUP1136, HUP1108, HUP1069, HUP1077, HUP1078, and HUP1109.

[Chem. 8]

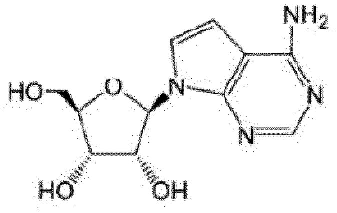

HUP1136

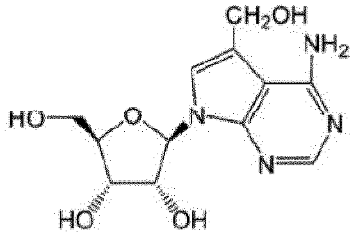

HUP1108

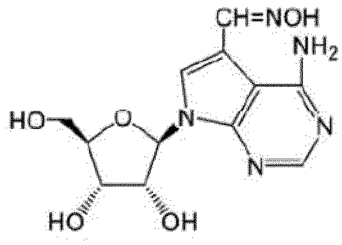

HUP1069

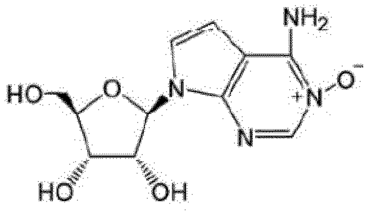

HUP1077

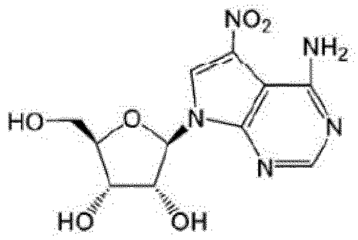

HUP1078

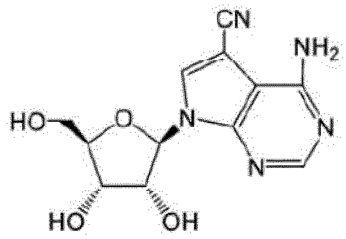

HUP1109

**4.** A pharmaceutical composition whose active ingredient is the antiviral agent of any among claims 1 to 3.

**5.** The pharmaceutical composition of claim 4, wherein the pharmaceutical composition is used to treat an infection due to a coronavirus or a flavivirus.

**6.** The pharmaceutical composition of claim 5, wherein the pharmaceutical composition is used to treat COVID-19.

**7.** The pharmaceutical composition of any one among claims 4 to 6, wherein the pharmaceutical composition is used for combination therapy with an inhibitor of 3CL protease.

**8.** The pharmaceutical composition of claim 7, wherein the inhibitor of 3CL protease is at least one type selected from the group consisting of PF-07304814 and S-217622.

**9.** A treatment method of treating a coronavirus infection or a flavivirus infection by administering an effective amount of the antiviral agent of any one among claims 1 to 3.

**10.** The treatment method of claim 9, wherein an effective amount of an antiviral agent composed of HUP1108 is administered.

**11.** The treatment method of claim 9 or 10, wherein the effective amount is less than 60 mg/kg.

**12.** The treatment method of claim 11, wherein the effective amount is 20 mg/kg.

**13.** The treatment method of claim 12, wherein the SARS-CoV-2 is at least one selected from the group consisting of WK-521 strain, $\alpha$ strain, $\beta$ strain, $\gamma$ strain, $\delta$ strain and o strain.

**14.** The treatment method of claim 13, wherein the SARS-CoV-2

**15.** The treatment method of any one of claims 9 to 12, wherein the flavivirus is at least one type selected from a group consisting of the dengue virus (DENV), the Zika virus (ZIKV), the yellow fever virus (YFV), the West Nile virus (WNV), and the Japanese encephalitis virus (JEV).

**16.** The treatment method of any one among claims 9 to 15, wherein an inhibitor of 3CL protease is administered in combination.

**17.** The treatment method of claim 17, wherein the inhibitor of the 3CL protease is at least one selected from the group consisting of PF-07304814 and S-217622.

**18.** A therapeutic combination of the antiviral agent of any one of claims 1 to 3 and an inhibitor of 3CL protease.

**19.** The combination of claim 18, wherein the inhibitor of the 3CL protease is at least one selected from the group consisting of PF-07304814 and S-217622.

**20.** The antiviral agent of any one among claims 1 to 3, wherein the antiviral agent is used to treat a coronavirus infection or a flavivirus infection.

**21.** The antiviral agent of claim 20, wherein the coronavirus is at least one type selected from a group consisting of SARS-CoV-2 WK-521 strain, $\alpha$ strain, $\beta$ strain, $\gamma$ strain, $\delta$ strain and o strain, and
the flavivirus is at least one type selected from a group consisting of the dengue virus (DENV), the Zika virus (ZIKV), the yellow fever virus (YFV), the West Nile virus (WNV), and the Japanese encephalitis virus (JEV).

[FIG. 1]

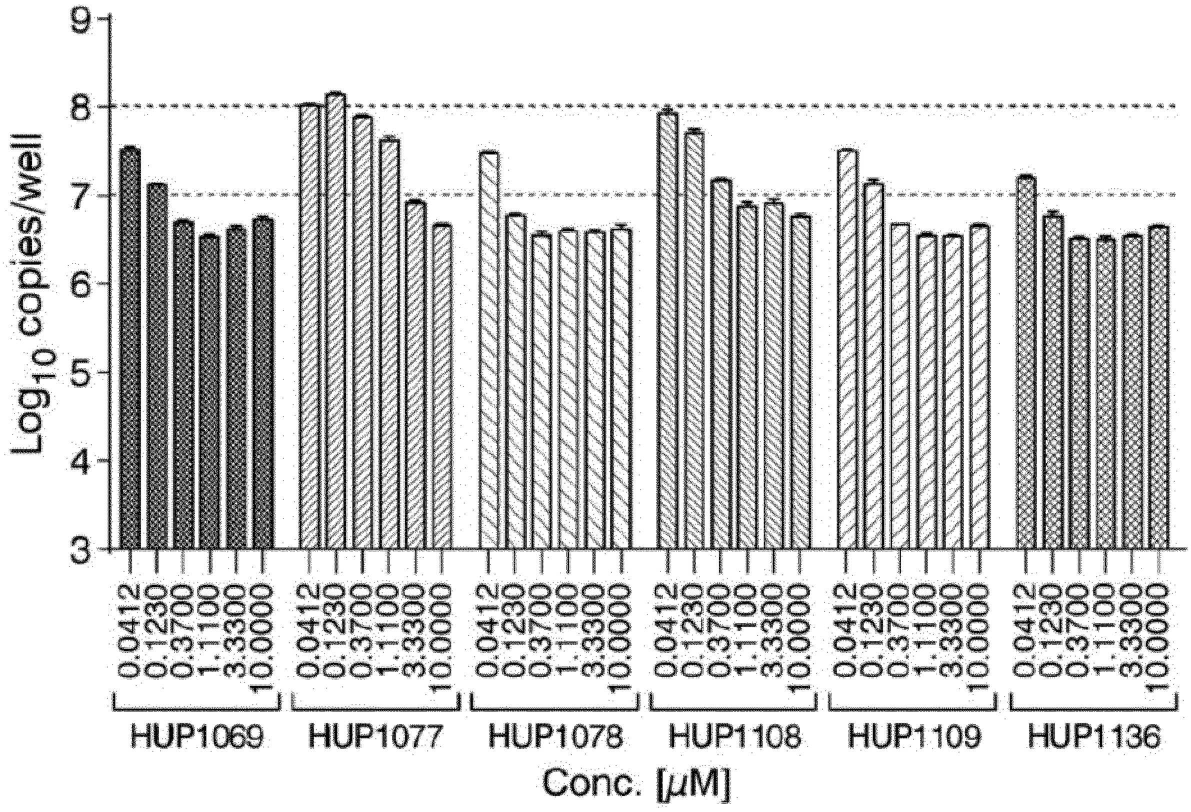
9
8
7
6
5
4
3
Log10 copies/well
0.0412
0.1230
0.3700
1.1100
3.3300
10.0000
HUP1069
HUP1077
HUP1078
HUP1108
HUP1109
HUP1136
Conc. [μM]

[FIG. 2]

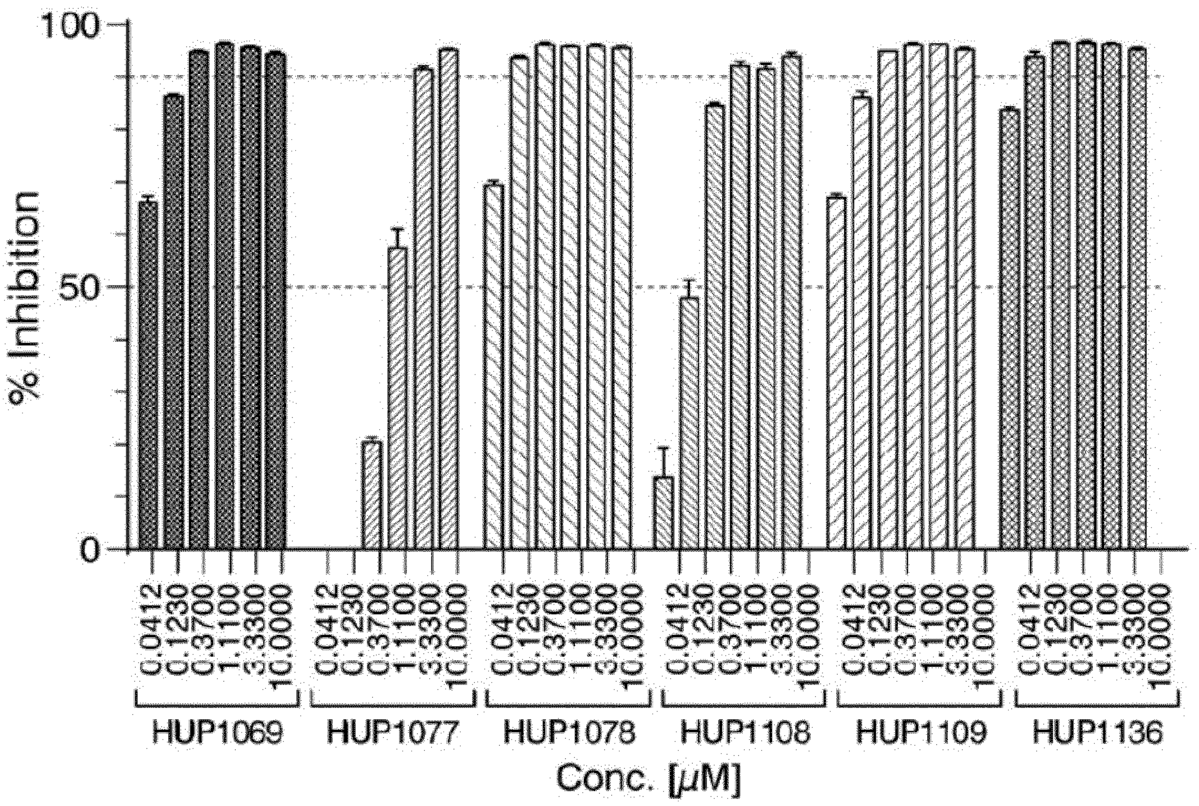
100
50
0
% Inhibition
0.0412
0.1230
0.3700
1.1100
3.3300
10.0000
HUP1069
HUP1077
HUP1078
HUP1108
HUP1109
HUP1136
Conc. [μM]

[FIG. 3]

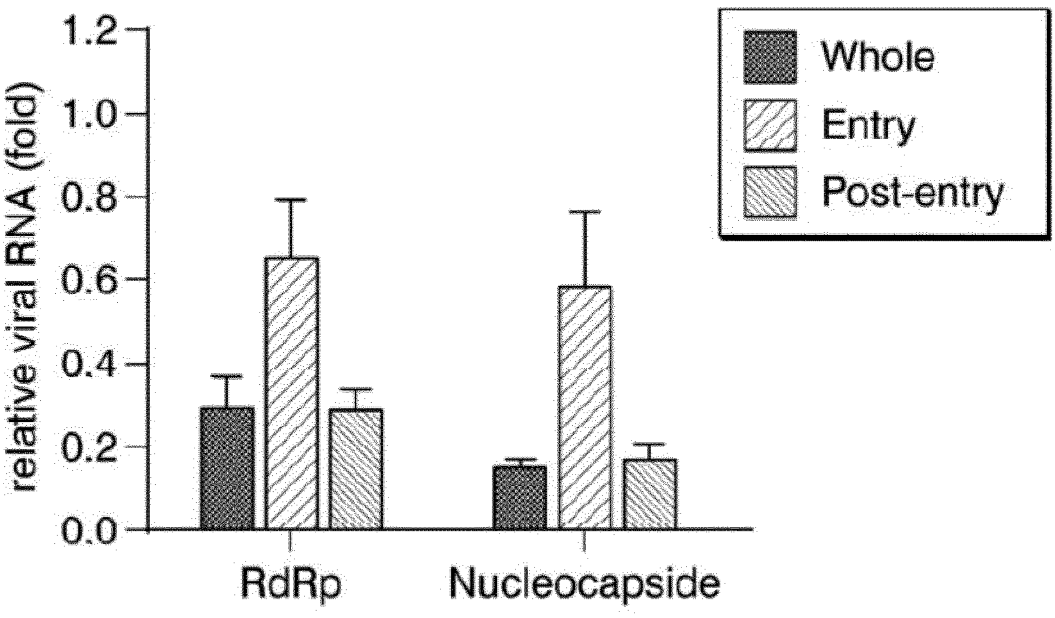
Whole
Entry
Post-entry
relative viral RNA (fold)
1.2
1.0
0.8
0.6
0.4
0.2
0.0
RdRp
Nucleocapside

[FIG. 4]

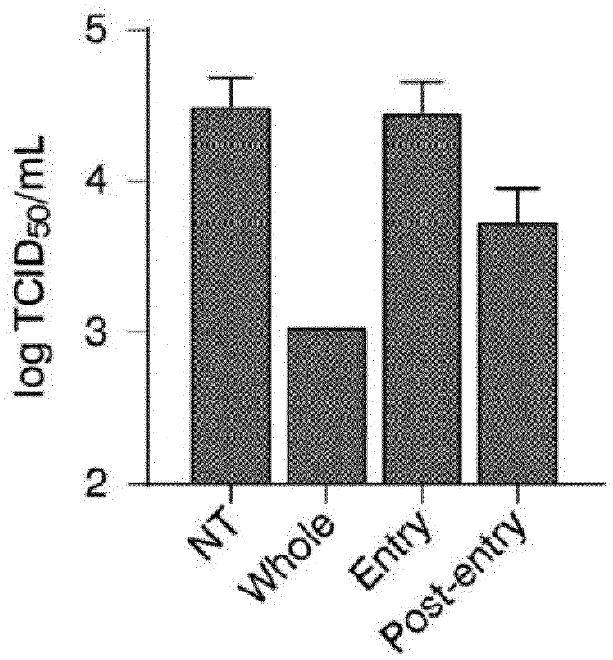
log TCID50/mL
5
4
3
2
NT
Whole
Entry
Post-entry

[FIG. 5]

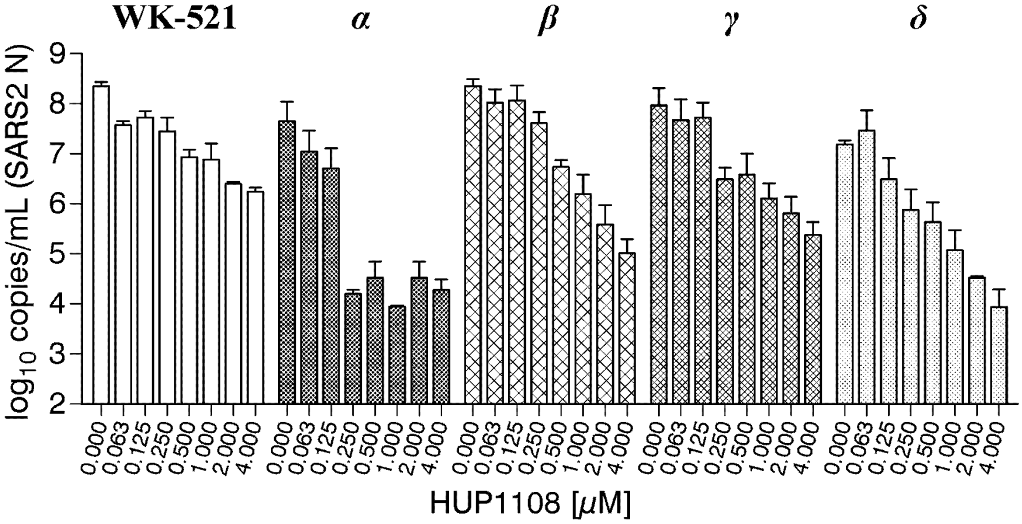


[FIG. 6]

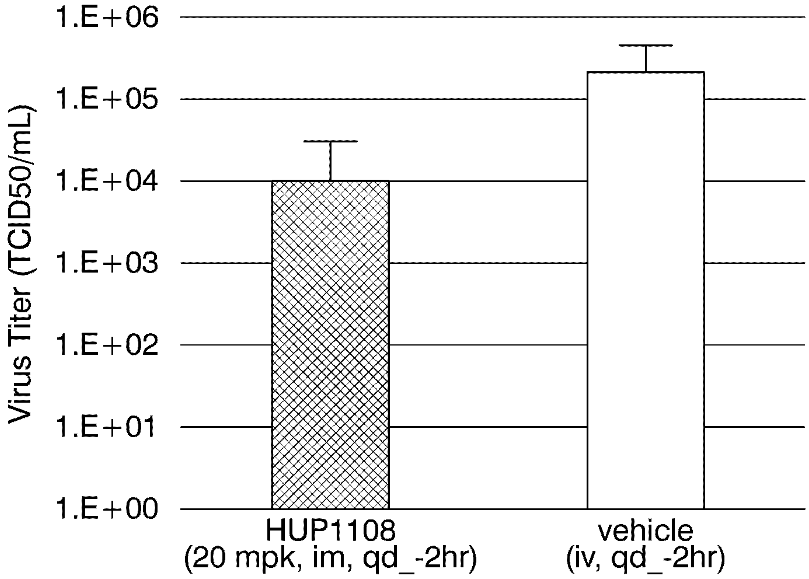


[FIG. 7]

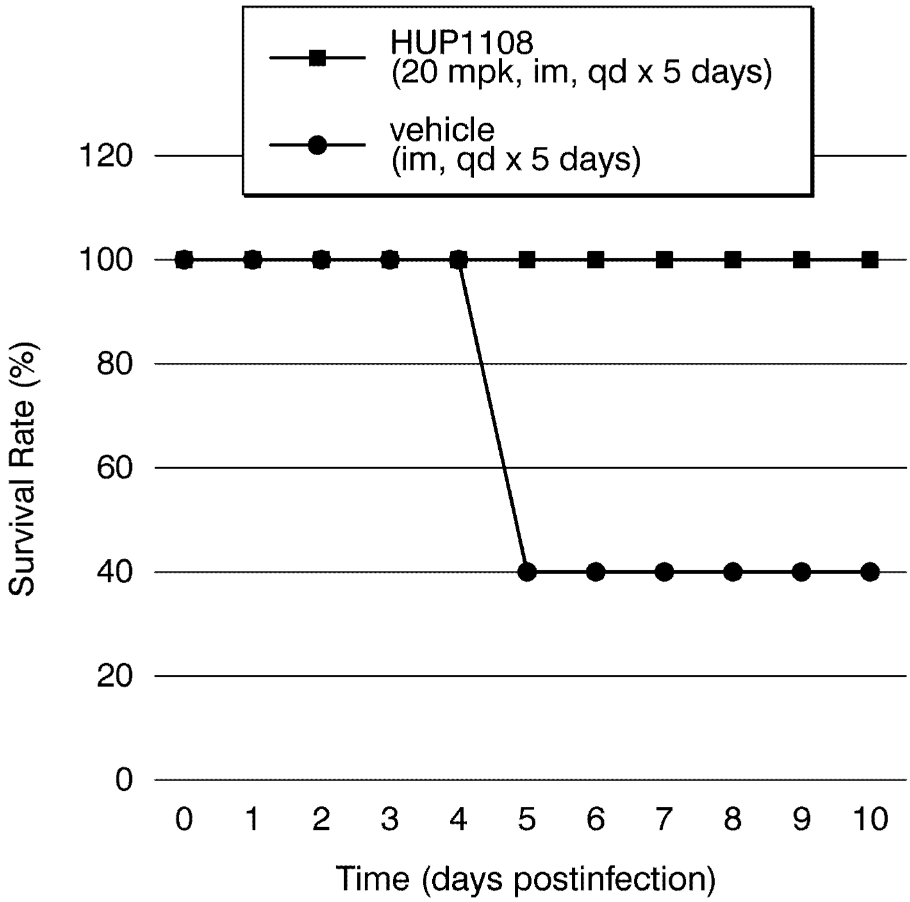
HUP1108
(20 mpk, im, qd x 5 days)
vehicle
(im, qd x 5 days)
Survival Rate (%)
120
100
80
60
40
20
0
0
1
2
3
4
5
6
7
8
9
10
Time (days postinfection)

## INTERNATIONAL SEARCH REPORT

International application No.
**PCT/JP2022/001997**

**A. CLASSIFICATION OF SUBJECT MATTER**

***A61K 31/7064***(2006.01)i; ***A61P 31/12***(2006.01)i
FI: A61K31/7064; A61P31/12

According to International Patent Classification (IPC) or to both national classification and IPC

**B. FIELDS SEARCHED**

Minimum documentation searched (classification system followed by classification symbols)

A61K31/7064; A61P31/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN), JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | ZHANG, Xuewu et al. Generation of predictive pharmacophore model for SARS-coronavirus main proteinase. European Journal of Medicinal Chemistry. 2005, vol. 40, no. 1, pp. 57-62, ISSN 0223-5234<br>in particular, abstract, p. 59, right column, p. 61, left column, table 3, fig. 3 | 1-5, 9, 11, 12, 20 |
| X | WU, Runzhi et al. Synthesis of a 6-Methyl-7-deaza Analogue of Adenosine That Potently Inhibits Replication of Polio and Dengue Viruses. J. Med. Chem. 2010, vol. 53, pp. 7958-7966, ISSN 0022-2623<br>in particular, p. 7958, chart 1, fig. 1, table 1 | 1-5, 9, 11, 12, 15, 20, 21 |
| X | BERGSTROM, Donald E. et al. Antiviral Activity of C-5 Substituted Tubercidin Analogues. J. Med. Chem. 1984, vol. 27, pp. 285-292, ISSN 0022-2623<br>in particular, compound no. 1, 8-11, chart 1, tables I-III | 1-4 |
| X | MONTGOMERY, John A. et al. Nucleoside analogs as antiviral agents. Biological Methylation and Drug Design. 1986, pp. 409-416<br>in particular, compound 13, p. 409, table 1 | 1-4 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 February 2022** | **08 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/001997**

**C. DOCUMENTS CONSIDERED TO BE RELEVANT**

| Category* | Citation of document, with indication, where appropriate, of the relevant passages | Relevant to claim No. |
|---|---|---|
| A | EYER, Ludek et al. Viral RNA-dependent RNA polymerase inhibitor 7-deaza-2'-C-methyladenosine prevents death in a mouse model of west nile virus infection. Antimicrobial Agents and Chemotherapy. 2019, vol. 63, no. 3, e02093-18, ISSN 1098-6596<br>in particular, table 1 | 1-21 |
| A | UEMATSU, Takayoshi et al. 5-Hydroxymethyltubercidin. Synthesis, biological activity, and role in pyrrolopyrimidine biosynthesis. Journal of Medicinal Chemistry. 1973, vol. 16, no. 12, pp. 1405-1407, ISSN 0022-2623<br>in particular, compounds 1-3, 5 | 1-21 |
| A | WO 2005/020885 A2 (ISIS PHRAMACEUTICALS, INC.) 10 March 2005 (2005-03-10)<br>in particular, claims, compounds 215, 565, 1223, 1285 | 1-21 |
| P, X | WO 2021/209993 A1 (YISSUM RESEARCH DEVELOPMENT COMPANY OF THE HEBREW UNIVERSITY OF JERUSALEM LTD.) 21 October 2021 (2021-10-21)<br>in particular, claims | 1-6, 9, 11-14, 20, 21 |
| P, X | WO 2021/234128 A1 (INSTITUT DE RECHERCHE EN SEMIOCHIMIE ET ETHOLOGIE APPLIQUEE) 25 November 2021 (2021-11-25)<br>in particular, claims, pp. 26-27 | 1-6, 9, 11-14, 20, 21 |
| P, X | WO 2021/158701 A1 (OYAGEN, INC.) 12 August 2021 (2021-08-12)<br>in particular, claims, examples | 1, 2, 4-6, 9, 11-14, 20, 21 |
| P, X | US 2021/0236497 A1 (OYAGEN, INC.) 05 August 2021 (2021-08-05)<br>in particular, claims, figures | 1, 2, 4-6, 9, 11-14, 20, 21 |
| P, A | WO 2021/205298 A1 (PFIZER INC.) 14 October 2021 (2021-10-14)<br>in particular, claims, example 49 | 1-21 |
| P, A | MCKIMM-BRESCHKIN, Jennifer L. et al. COVID-19, Influenza and RSV: Surveillance-informed prevention and treatment - Meeting report from an isirv-WHO virtual conference. Antiviral Research. 2022, vol. 197, 105227, Available Online 18 December 2021<br>in particular, abstract, 5.3 S-217622 a potential oral SARS-CoV-2 3C like protease inhibitor | 1-21 |

Form PCT/ISA/210 (second sheet) (January 2015)

**INTERNATIONAL SEARCH REPORT**
**Information on patent family members**

International application No.
**PCT/JP2022/001997**

| Patent document cited in search report | Publication date (day/month/year) | Patent family member(s) | Publication date (day/month/year) |
|---|---|---|---|
| WO 2005/020885 A2 | 10 March 2005 | (Family: none) | |
| WO 2021/209993 A1 | 21 October 2021 | (Family: none) | |
| WO 2021/234128 A1 | 25 November 2021 | (Family: none) | |
| WO 2021/158701 A1 | 12 August 2021 | (Family: none) | |
| US 2021/0236497 A1 | 05 August 2021 | (Family: none) | |
| WO 2021/205298 A1 | 14 October 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **YAN ; MULLER.** *ACS Medicinal Chemistry Letters,* 2020, vol. 11, 1361-1366
- **WATANABE ; UEDA.** *Nucleosides & Nucleotides,* 1983, vol. 2 (2), 113-125
- **BERGSTROM et al.** *Journal of Organic Chemistry,* 1981, vol. 46 (7), 1423-1431
- **UEMATSU ; SUHADOLNIK.** *Journal of Medicinal Chemistry,* 1973, vol. 16 (12), 1405-1407
- **CHO et al.** *Bioorganic & Medicinal Chemistry Letters,* 2012, vol. 22, 2705-2707
- **SCHAFER et al.** *bioRxiv preprint*
- **RASMUSSEN et al.** *Viruses,* 2021, vol. 13 (7), 1369